(19) Europäisches Patentamt
European Patent Office
Office européen des brevets

(11) EP 2 272 860 A1

(12) EUROPEAN PATENT APPLICATION

(43) Date of publication:
12.01.2011 Bulletin 2011/02

(51) Int Cl.:
*C07K 14/18* (2006.01) *G01N 33/543* (2006.01)
*G01N 33/68* (2006.01)

(21) Application number: 09290560.3

(22) Date of filing: 10.07.2009

(84) Designated Contracting States:
AT BE BG CH CY CZ DE DK EE ES FI FR GB GR HR HU IE IS IT LI LT LU LV MC MK MT NL NO PL PT RO SE SI SK SM TR
Designated Extension States:
AL BA RS

(71) Applicant: Institut Pasteur
75015 Paris (FR)

(72) Inventors:
- Bedouelle, Hugues
75015 Paris (FR)
- Dussart, Philippe
97351 Matoury (GF)
- Zidane, Nora
75015 Paris (FR)
- Bremand, Laëtitia
97300 Cayenne (GF)

(74) Representative: Thomas, Dean et al
Cabinet Ores
36 rue de St Pétersbourg
75008 Paris (FR)

(54) **Multivalent epitope in complex with a detection marker for the early serodiagnosis of infections**

(57) The present invention relates to a new method to determine the presence of antibodies to a pathogen in a serological sample using a new detection reagent which comprises at least two and preferably at least four copies of an antigen from the pathogen and a detectable marker. The present invention also relates to a new detection reagent which consists of at least two and preferably at least four antigenic peptides in a complex with a detectable marker via interacting multimerization domains upon both the antigenic peptides and detectable marker.

EP 2 272 860 A1

SEQUENCE LISTING

<110> Institut Pasteur

<120> Multivalent epitope in complex with a detection marker for the early serodiagnosis of infections

<130> F226 - 172 EP

<160> 10

<170> PatentIn version 3.3

<210> 1
<211> 460
<212> DNA
<213> Artificial

<220>
<223> Coding seq for AviTag-ED3.Den1-H6

<400> 1
```
atggctggtc tgaacgatat cttcgaagct cagaaaatcg aatggcacga aggttccatg      60
gggatgtcat atgtgatgtg cacaggctca tttaagctag agaaggaagt ggctgagacc     120
cagcatggga ctgtcctagt gcaggttaaa tacgaaggaa cagatgcgcc atgcaagatc     180
ccctttttcga cccaagatga gaaaggagtg acccagaatg ggagattgat aacagccaat    240
cccatagtta ctgacaaaga aaaaccagtc aacattgaga cagaaccacc ttttggtgag     300
agctacatca tagtaggggc aggtgaaaaa gctttgaaac taagctggtt caagaaggga     360
agcagcatag ggaaactcga gcaccaccac caccaccact gagatccggc tgctaacaaa     420
gcccgaaagg aagctgagtt ggctgctgcc accgctgagc                           460
```

<210> 2
<211> 133
<212> PRT
<213> Artificial

<220>
<223> Peptide Seq for AviTag-ED3.Den1-H6

<400> 2

```
Met Ala Gly Leu Asn Asp Ile Phe Glu Ala Gln Lys Ile Glu Trp His
1               5                   10                  15

Glu Gly Ser Met Gly Met Ser Tyr Val Met Cys Thr Gly Ser Phe Lys
            20                  25                  30

Leu Glu Lys Glu Val Ala Glu Thr Gln His Gly Thr Val Leu Val Gln
        35                  40                  45

Val Lys Tyr Glu Gly Thr Asp Ala Pro Cys Lys Ile Pro Phe Ser Thr
    50                  55                  60

Gln Asp Glu Lys Gly Val Thr Gln Asn Gly Arg Leu Ile Thr Ala Asn
65                  70                  75                  80
```

Pro Ile Val Thr Asp Lys Glu Lys Pro Val Asn Ile Glu Thr Glu Pro
85 90 95

Pro Phe Gly Glu Ser Tyr Ile Ile Val Gly Ala Gly Glu Lys Ala Leu
100 105 110

Lys Leu Ser Trp Phe Lys Lys Gly Ser Ser Ile Gly Lys Leu Glu His
115 120 125

His His His His His
130

<210> 3
<211> 160
<212> PRT
<213> Streptomyces avidinii

<400> 3

Met Asp Pro Ser Lys Asp Ser Lys Ala Gln Val Ser Ala Ala Glu Ala
1 5 10 15

Gly Ile Thr Gly Thr Trp Tyr Asn Gln Leu Gly Ser Thr Phe Ile Val
20 25 30

Thr Ala Gly Ala Asp Gly Ala Leu Thr Gly Thr Tyr Glu Ser Ala Val
35 40 45

Gly Asn Ala Glu Ser Arg Tyr Val Leu Thr Gly Arg Tyr Asp Ser Ala
50 55 60

Pro Ala Thr Asp Gly Ser Gly Thr Ala Leu Gly Trp Thr Val Ala Trp
65 70 75 80

Lys Asn Asn Tyr Arg Asn Ala His Ser Ala Thr Thr Trp Ser Gly Gln
85 90 95

Tyr Val Gly Gly Ala Glu Ala Arg Ile Asn Thr Gln Trp Leu Leu Thr
100 105 110

Ser Gly Thr Thr Glu Ala Asn Ala Trp Lys Ser Thr Leu Val Gly His
115 120 125

Asp Thr Phe Thr Lys Val Lys Pro Ser Ala Ala Ser Ile Asp Ala Ala
130 135 140

Lys Lys Ala Gly Val Asn Asn Gly Asn Pro Leu Asp Ala Val Gln Gln
145 150 155 160

<210> 4
<211> 133
<212> PRT
<213> Artificial

<220>
<223> Peptide Seq for AviTag-ED3.Den2-H6

<400> 4

```
Met Ala Gly Leu Asn Asp Ile Phe Glu Ala Gln Lys Ile Glu Trp His
1               5                   10                  15

Glu Gly Ser Met Gly Met Ser Tyr Ser Met Cys Thr Gly Lys Phe Lys
            20                  25                  30

Ile Val Lys Glu Ile Ala Glu Thr Gln His Gly Thr Ile Val Ile Arg
        35                  40                  45

Val Gln Tyr Glu Gly Asp Gly Ser Pro Cys Lys Ile Pro Phe Glu Ile
    50                  55                  60

Met Asp Leu Glu Lys Arg His Val Leu Gly Arg Leu Ile Thr Val Asn
65                  70                  75                  80

Pro Ile Val Thr Glu Lys Asp Ser Pro Val Asn Ile Glu Ala Glu Pro
                85                  90                  95

Pro Phe Gly Asp Ser Tyr Ile Ile Ile Gly Val Glu Pro Gly Gln Leu
            100                 105                 110

Lys Leu Asn Trp Phe Lys Lys Gly Ser Ser Ile Gly Gln Leu Glu His
        115                 120                 125

His His His His His
    130
```

<210> 5
<211> 133
<212> PRT
<213> Artificial

<220>
<223> Peptide Seq for AviTag-ED3.Den3-H6

<400> 5

```
Met Ala Gly Leu Asn Asp Ile Phe Glu Ala Gln Lys Ile Glu Trp His
1               5                   10                  15

Glu Gly Ser Met Gly Met Ser Tyr Ala Met Cys Leu Asn Thr Phe Val
            20                  25                  30

Leu Lys Lys Glu Val Ser Glu Thr Gln His Gly Thr Ile Leu Ile Lys
        35                  40                  45

Val Glu Tyr Lys Gly Glu Asp Ala Pro Cys Lys Ile Pro Phe Ser Thr
    50                  55                  60
```

Glu Asp Gly Gln Gly Lys Ala His Asn Gly Arg Leu Ile Thr Ala Asn
65 70 75 80

Pro Val Val Thr Lys Lys Glu Glu Pro Val Asn Ile Glu Ala Glu Pro
85 90 95

Pro Phe Gly Glu Ser Asn Ile Val Ile Gly Ile Gly Asp Lys Ala Leu
100 105 110

Lys Ile Asn Trp Tyr Lys Lys Gly Ser Ser Ile Gly Lys Leu Glu His
115 120 125

His His His His His
130

<210> 6
<211> 133
<212> PRT
<213> Artificial

<220>
<223> Peptide Seq for AviTag-ED3.Den4-H6

<400> 6

Met Ala Gly Leu Asn Asp Ile Phe Glu Ala Gln Lys Ile Glu Trp His
1 5 10 15

Glu Gly Ser Met Gly Met Ser Tyr Thr Met Cys Ser Gly Lys Phe Ser
20 25 30

Ile Asp Lys Glu Met Ala Glu Thr Gln His Gly Thr Thr Val Val Lys
35 40 45

Val Lys Tyr Glu Gly Ala Gly Ala Pro Cys Lys Val Pro Ile Glu Ile
50 55 60

Arg Asp Val Asn Lys Glu Lys Val Val Gly Arg Ile Ile Ser Ser Thr
65 70 75 80

Pro Phe Ala Glu Asn Thr Asn Ser Val Thr Asn Ile Glu Leu Glu Pro
85 90 95

Pro Phe Gly Asp Ser Tyr Ile Val Ile Gly Val Gly Asp Ser Ala Leu
100 105 110

Thr Leu His Trp Phe Arg Lys Gly Ser Ser Ile Gly Lys Leu Glu His
115 120 125

His His His His His
130

<210> 7
<211> 136

<212> PRT
<213> Artificial

<220>
<223> Peptide Seq for AviTag-ED3.WNV-H6

<400> 7

Met Ala Gly Leu Asn Asp Ile Phe Glu Ala Gln Lys Ile Glu Trp His
1 5 10 15

Glu Gly Ser Met Gly Thr Thr Tyr Gly Val Cys Ser Lys Ala Phe Lys
20 25 30

Phe Leu Gly Thr Pro Ala Asp Thr Gly His Gly Thr Val Val Leu Glu
35 40 45

Leu Gln Tyr Thr Gly Thr Asp Gly Pro Cys Lys Val Pro Ile Ser Ser
50 55 60

Val Ala Ser Leu Asn Asp Leu Thr Pro Val Gly Arg Leu Val Thr Val
65 70 75 80

Asn Pro Phe Val Ser Val Ala Thr Ala Asn Ala Lys Val Leu Ile Glu
85 90 95

Leu Glu Pro Pro Phe Gly Asp Ser Tyr Ile Val Val Gly Arg Gly Glu
100 105 110

Gln Gln Ile Asn His His Trp His Lys Ser Gly Ser Ser Ile Gly Lys
115 120 125

Leu Glu His His His His His His
130 135

<210> 8
<211> 138
<212> PRT
<213> Artificial

<220>
<223> Peptide Seq for AviTag-ED3.JEV-H6

<400> 8

Met Ala Gly Leu Asn Asp Ile Phe Glu Ala Gln Lys Ile Glu Trp His
1 5 10 15

Glu Gly Ser Met Ala Met Gly Thr Thr Tyr Gly Met Cys Thr Glu Lys
20 25 30

Phe Ser Phe Ala Lys Asn Pro Ala Asp Thr Gly His Gly Thr Val Val
35 40 45

Ile Glu Leu Ser Tyr Ser Gly Ser Asp Gly Pro Cys Lys Ile Pro Ile
50 55 60

Val Ser Val Ala Ser Leu Asn Asp Met Thr Pro Val Gly Arg Leu Val
65 70 75 80

Thr Val Asn Pro Phe Val Ala Thr Ser Ser Ala Asn Ser Lys Val Leu
85 90 95

Val Glu Met Glu Pro Pro Phe Gly Asp Ser Tyr Ile Val Val Gly Arg
100 105 110

Gly Asp Lys Gln Ile Asn His His Trp His Lys Ala Gly Ser Thr Leu
115 120 125

Gly Lys Leu Glu His His His His His His
130 135

<210> 9
<211> 133
<212> PRT
<213> Artificial

<220>
<223> Peptide Seq for AviTag-ED3.YFV-H6

<400> 9

Met Ala Gly Leu Asn Asp Ile Phe Glu Ala Gln Lys Ile Glu Trp His
1 5 10 15

Glu Gly Ser Met Gly Thr Ser Tyr Lys Ile Cys Thr Asp Lys Met Phe
20 25 30

Phe Val Lys Asn Pro Thr Asp Thr Gly His Gly Thr Val Val Met Gln
35 40 45

Val Lys Val Ser Lys Gly Ala Pro Cys Arg Ile Pro Val Ile Val Ala
50 55 60

Asp Asp Leu Thr Ala Ala Ile Asn Lys Gly Ile Leu Val Thr Val Asn
65 70 75 80

Pro Ile Ala Ser Thr Asn Asp Asp Glu Val Leu Ile Glu Val Asn Pro
85 90 95

Pro Phe Gly Asp Ser Tyr Ile Ile Val Gly Arg Gly Asp Ser Arg Leu
100 105 110

Thr Tyr Gln Trp His Lys Glu Gly Ser Ser Ile Gly Lys Leu Glu His
115 120 125

His His His His His
130

```
<210>  10
<211>  136
<212>  PRT
<213>  Artificial

<220>
<223>  Peptide Seq for AviTag-ED3.SLEV-H6

<400>  10

Met Ala Gly Leu Asn Asp Ile Phe Glu Ala Gln Lys Ile Glu Trp His
1               5                   10                  15

Glu Gly Ser Met Gly Thr Thr Tyr Gly Met Cys Asp Ser Ala Phe Thr
            20                  25                  30

Phe Ser Lys Asn Pro Thr Asp Thr Gly His Gly Thr Val Ile Val Glu
        35                  40                  45

Leu Gln Tyr Thr Gly Ser Asn Gly Pro Cys Arg Val Pro Ile Ser Val
    50                  55                  60

Thr Ala Asn Leu Met Asp Leu Thr Pro Val Gly Arg Leu Val Thr Val
65                  70                  75                  80

Asn Pro Phe Ile Ser Thr Gly Gly Ala Asn Asn Lys Val Met Ile Glu
                85                  90                  95

Val Glu Pro Pro Phe Gly Asp Ser Tyr Ile Val Val Gly Arg Gly Thr
            100                 105                 110

Thr Gln Ile Asn Tyr His Trp His Lys Glu Gly Ser Ser Ile Gly Lys
        115                 120                 125

Leu Glu His His His His His His
    130                 135
```

## Description

The present invention relates to a new method to determine the presence of antibodies to a pathogen in a serological sample using a new detection reagent which comprises at least two and preferably at least four copies of an antigen from the pathogen and a detectable marker. The present invention also relates to a new detection reagent which consists of at least two and preferably at least four antigenic peptides in a complex with a detectable marker via interacting portions upon both the antigenic peptides and detectable marker.

In order to best treat a disease or illness afflicting a patient it is necessary to determine as soon as possible the causative agent of the disease. In the case of illnesses caused by infective agents such as viruses, bacteria or protozoa a further problem is the identification of the particular infective agent, as in general the infection of a patient gives rise to certain common symptoms.

Febrile illnesses are characterised by a period of fever which can last a short time, continue throughout the infection or arise intermittently whilst the infection persists. Within the group of febrile illnesses are a number of 'low' risk illnesses, that is illnesses which generally do not have any long term effects upon the patients health, such as the common cold, measles and chicken pox; and 'high' risk diseases, that is diseases which often lead to patient mortality or severe symptoms which prevent the patient from recovering to their original level of health. 'High' risk febrile diseases include severe acute respiratory syndrome (SARS), typhoid fever as well as the group of diseases caused by flaviviruses such as dengue hemorrhagic fever (DHF) and dengue shock syndrome (DSS).

This commonality of early symptoms can make the accurate diagnosis of the agent of disease very difficult if not impossible using directly measurable or observable symptoms. The resulting delay in diagnosis can in turn retard the deployment of an effective treatment regime for the patients' disease and lead to a reduction in the speed and in some cases chances of the patient recovering.

It is also currently difficult to make an early and definitive diagnosis using more advanced techniques such as a serological analysis of reacting antibodies for the pathogen or the direct detection of the pathogen by for instance RT-PCR. The reasons for this vary between pathogens but in general most existing serological techniques exhibit non-specific cross reactions between antibodies with non-target antigens or limitations in terms of when a PCR assay can lead to a meaningful result. Further problems include the inability using current assays to differentiate between a primary and secondary (or further) infection by the same pathogen and also the time necessary to perform the assay. The determination of whether an infection is the first or a subsequent re-infection can be of critical importance as certain pathogens are known to induce more pathogenic reactions upon subsequent infection due in part to the altered nature of the patients' immune response.

To illustrate the problems detailed above the important group of disease causing viruses, flaviviruses, will be discussed in detail together with an analysis of their epidemiology and the current problems with making an early diagnosis so that an appropriate treatment regime can be deployed.

Flaviviruses are enveloped RNA viruses. They encode three structural proteins, the core protein C, the premembrane/membrane protein prM/M and the envelope glycoprotein gpE, and seven non-structural (NS) proteins. The structures of the whole virus and of gpE have been solved for several flaviviruses by electron cryomicroscopy and X-ray crystallography. Ninety dimers of gpE cover the surface of the virus. Each monomer comprises three ectodomains, ED1 to ED3, and a transmembrane segment. ED1 and ED2 are interwoven. ED2 contains the dimerization interface and the peptide of fusion with the cellular membrane. ED3 is continuous and comprises residues 296-400 of gpE. Its fold is compact, immunoglobulin-like and stabilized by a disulfide bond (Mukhopadhyay et al. 2005).

Many flaviviruses are responsible for widespread emerging or re-emerging diseases, e.g. the four serotypes of dengue virus (DENV1 to DENV4), the West-Nile virus (WNV), the yellow fever virus (YFV), and the Japanese encephalitis virus (JEV). Infections with flaviviruses cause three main clinical syndromes in humans: systemic febrile illness, hemorrhagic fever and meningoencephalitis. The illness generally follows a biphasic course with a mild febrile phase, often going unrecognized, followed by defervescence and an apparent relapse about five to seven days after the onset of symptoms, and then by a more severe illness in a few percent of cases (Gould and Solomon 2008). These syndromes are shared with other diseases, including measles, typhoid fever, leptospiroses and severe acute respiratory syndrome (SARS) and their co-occurence in the same area makes a specific diagnosis difficult (Potts and Rothman 2008).

The 'high' risk illnesses that are caused by DENV, include the dengue hemorrhagic fever DHF and shock syndrome DSS (WHO 1997). The high mortality associated with DHF and DSS (40 to 50 % for untreated patients) can be minimized through timely medical care and fluid replacement, which depend on accurate diagnosis (Igarashi 1997). Infections by DENV result in the production of neutralizing antibodies and lifelong immunity against the homologous DENV serotype but there is no cross-immunity against the heterologous serotypes. Moreover, secondary infection is suspected to be a risk factor for DHF and DSS (Halstead 1988; Mathew and Rothman 2008).

Flavivirus and in particular DENV infections can be diagnosed during the acute phase of the disease by virus isolation, RT-PCR of viral RNA, or immunochemical detection of the viral NS1 protein in serum samples (Kuno 2003; Shu and Huang 2004; Dauphin and Zientara 2007). However, these methods can only be used during the viremic phase of infection, i.e. 4-6 days after the onset of symptoms and they no longer work after defervescence (Yamada et al. 1999; de Oliveira Poersch et al. 2005; Dussart et al. 2006). Moreover, these techniques cannot distinguish between primary and secondary infections (Schilling et al. 2004).

The kinetics of the immune response to a flaviviral infection have been well characterized for dengue. In primary infections, immunoglobulin M (IgM) antibodies that are directed against DENV, appear as early as 4-6 days after the onset of symptoms, peak at about 2 weeks, and wane thereafter. IgG antibodies appear shortly afterwards and persist for several years. In secondary infections, IgMs appear earlier than in primary infections, often with lower titers, whereas IgGs appear with high titers either before or along with IgMs (Innis et al. 1989; Kuno 2003; Schilling et al. 2004; Shu and Huang 2004; Teles et al. 2005; Dauphin and Zientara 2007).

Cross-reactive antibodies are produced during an infection with a flavivirus. They react with the infecting virus but also with other flaviviruses, *Flaviviridae* or even unrelated infectious agents. These cross-reactive antibodies give rise to false positives in serological tests, e.g. enzyme-linked immunosorbent assays (ELISA) and complicate their interpretation. For example, cross-reactions arise between sera from patients infected with DENV or vaccinated against YFV and JEV, when whole virus antigens are used in the tests. They also arise between sera from patients with flaviviral diseases and those from patients with malaria, typhoid, leptospirosis or autoimmune diseases (Schwartz et al. 2000; Takasaki et al. 2002; Kuno 2003; Mongkolsapaya et al. 2003; AnandaRao et al. 2005; Holmes et al. 2005).

Generally, IgMs are less cross-reactive than IgG but they have a lower affinity for their antigen than IgGs since they do not undergo somatic hyper-mutagenesis (Westaway et al. 1975; Makino et al. 1994; Martin et al. 2002; Kuno 2003; Dauphin and Zientara 2007). In IgM-specific indirect ELISA, see below, the IgM signal can be quenched by the high amount of non-specific IgG present in the serum. In IgM-antibody specific capture ELISA (MAC-ELISA) see below, the IgMs of the serum are first captured by anti-IgM antibodies. MAC-ELISAs are therefore more sensitive and specific than IgM-specific indirect ELISAs and preferred over them (Kuno 2003).

Current diagnostic assays for flavivirus infections utilize either ELISA or dipstick formats for the identification of flavivirus infections.

The immunosorbent assays (ISA) for the detection of viral antibodies in the serum of patients belong to two main types: indirect ISA and antibody-specific capture ISA.

In an indirect ISA, a solid support is sensitized with the viral antigen (virAg). The immobilized antigen is reacted with the human or animal serum under analysis. Finally, the bound antibodies are revealed with a reporter system, which generally consists of a conjugate between an immunoglobulin binding protein (@Ig) and an enzyme (Enz), typically horseradish peroxidase (HRP) or alkaline phosphatase (PhoA). This being an enzyme-linked ISA (ELISA). Other types of probes can be used, e.g. a fluorophore or colloidal gold. The general scheme for an indirect ISA is the following:

$$\text{Support-virAg :: Serum :: @Ig-Reporter} \qquad (1)$$

where "-" stands for a covalent bond or immobilization; and "::", for non-covalent interactions. The Ig binding protein may be specific for a particular class of Ig (@IgX, where X = M, A or G). In that case, one speaks of an IgX-specific indirect ISA. Several variations of the indirect ISA have been described, in particular the antigen capture ISA, the epitope blocking ISA, and the avidity ISA (Blitvich et al., 2003; Johnson et al., 2000; Matheus et al., 2005).

In an IgM specific capture ISA (MAC-ELISA), a solid support is sensitized with an Ig binding protein (@IgX, with X = M, A (AAC-ELISA) or G (GAC-ELISA), which is directed against a specific class of Ig molecules of the animal species under consideration and most generally consists of heterologous antibodies. The immobilized Ig binding protein is reacted successively with the serum under analysis, the viral antigen and then a reporter system, which generally consists of a conjugate between an antigen binding molecule (@virAg) and an enzyme (Enz). A generic IgX specific capture ISA (XAC-ISA) can be schematized as follows:

$$\text{Support-@IgX :: Serum :: virAg :: @virAg-Reporter} \qquad (2)$$

Depending on the Ig binding protein (@IgX), one can speak of IgM, IgG or IgA antibody capture immunosorbent assays (MAC-ELISA, GAC-ELISA or AAC-ELISA).

The immunosorbent assays for IgM antibodies are among the most useful serologic procedures for determining recent infections by flaviviruses, since these IgM molecules appear early in infection, rise rapidly in the course of the disease, and are usually less cross-reactive with other viruses than IgG antibodies (Kuno, 2003). IgM molecules can be detected as soon as the 5th day after infection but their affinity for a monomeric antigen is generally lower than that of other immunoglobulin molecule types.

The MAC-ELISA is preferred over the IgM-specific indirect ELISA because the IgG antibodies from previous infections by related viruses can have a suppressive effect on the sensitivity of the latter assay (Vorndam and Kuno, 1997). It is recommended by WHO for the serological diagnosis of several flaviviral infections and in particular dengue (WHO, 1997).

The MAC-ELISA has the following advantages: If paired serum samples are available, a rising, stable or falling titer in IgM can indicate the time of infection. The ratio of IgM to IgG antibody in parallel MAC- and GAC-ELISA on a single sample can be used to differentiate primary from secondary infections since the IgM/IgG ratio is higher than one in the former case and lower in the latter (Innis et al., 1989). It can detect anti-flaviviral IgM in the cerebrospinal fluid and saliva (Kao et al., 2005; Teles et al., 2005). IgA specific ELISAs have also been developed. The IgA response develops after the IgM response but before the IgG one. The IgA/IgM ratio in parallel MAC- and AAC-ELISAs can indicate whether the infection is recent or dates from a few months, for DENV and WNV (Prince and Lape-Nixon, 2005; Talarmin et al., 1998).

The specificity of the immunosorbent assays comes mainly from the interaction between the serum under analysis and the antigen and thus depends on the nature of the antigen preparation. However, it may also come from the nature of the reporter molecule.

Other types of ISA exist such as <u>sandwich ELISA</u> (R. J. Kerschbaumer et al., 1996) whose format is the following:

$$\text{Support-@GST :: GST-(3D6 epitope) :: scFv3D6-PhoA} \qquad (3)$$

where @GST represents antibodies directed against the Glutathione-S-Transferase (GST); GST-(3D6 epitope), a hybrid protein between GST and an epitope of antibody 3D6; and scFv3D6-PhoA, a hybrid protein between a single-chain variable fragment (scFv) of antibody 3D6 and alkaline phosphatase.

Sandwich ELISA assays are used to detect the presence of an antigen in the serum of patients, but not antibodies directed against an infectious agent as in the current invention. Moreover such methods require the isolation and characterization of at least two non-competing antibodies to be used in the assay.

Another type of ISA is a <u>reverse ELISA</u> (D. Ludolfs et al., 2007) whose format is the following:

$$\text{Support-RF :: serum :: rED3-HRP} \qquad (4)$$

where the Rhumatoid Factor (RF) is an autoimmune antibody that recognizes the Fc fragment of the IgG immunoglobulins; and rED3-HRP is a chemical conjugate between Horseradish Peroxydase (HRP) and a recombinant domain 3 (rED3) of the envelope protein E from the West-Nile virus. HRP is a monomeric protein, whereas alkaline phosphatase is dimeric, and the rED3-HRP hybrid protein was obtained by chemical coupling of the two partners, rED3 and HRP.

Until recently, the antigens used for ISA were mainly extracts of suckling mouse brains (SMB) or cell cultures, infected by the virus under consideration. These are being progressively replaced with recombinant prM/gpE virus like particles (VLP), where prM and gpE are the precursor of the membrane protein and envelope glycoprotein of the virus or with a recombinant extracellular domain (sE) of gpE. The non-structural protein NS1 has also been used as an antigen in both IgG-specific indirect ELISA and MAC-ELISA. NS1 can differentiate between primary and secondary infections and correctly identify the serotype of the infecting DENV in the sera of patients with primary infection (Shu et al., 2004; Shu et al., 2003; Shu et al., 2002).

Previously (Bedouelle et al., 2008, WO2008/152528), the inventors have created a new class of reagent for use in ELISA method to detect antiflavivirus antibodies. This reagent consisted of a hexahistidine, the ED3 domain from a flavivirus and alkaline phosphatase from *E. coli* (H6-ED3-PhoA) in a single peptide. This reagent is an obligate dimer. In this way, the inventors generated a first class of reagents that can be used to detect lower affinity antibodies through an avidity effect and in particular IgM antibodies.

These dimeric reagents $(\text{H6-ED3-PhoA})_2$ validated the concept of using an antigenic viral domain, and in particular the ED3 domain from flaviviruses, in a bivalent state, to detect specific low affinity IgMs in the serum of patients by MAC-ELISA.

Serious problems still exist however with the detection of Flaviviruses and other pathogens using serological assays even using the new class of reagent developed by the inventors and these include the specificity of the assay. Although the dimeric reagents were shown to have much improved performance with respect to prior art techniques and in particular allowed the differentiation of DENV serotypes 1, 2 and 3 they were still unable to distinguish DENV4. In addition due to the levels of affinity between the bivalent reagent and its antibody targets a minimum of 3 hours was required for the chromogenic reaction (Bedouelle et al., 2008, WO2008/152528). These limitations also make it difficult to determine whether an infection is a primary or secondary infection by the pathogen. Finally the use of the *E. coli* PhoA limits these reagents as many immunoenzymatic diagnostic assays are based on horseradish peroxidase (HRP) rather than PhoA because the former has a higher activity and is smaller, more stable, and cheaper to produce than the latter. The inventors used the PhoA enzyme as a marker as it can be easily expressed as a single peptide in combination with the selected antigen. It is not possible to prepare a HRP/ED3 single antigenic peptide as ED3 requires specific conditions so as to allow the formation of an essential disulfide bond which in turn would render the HRP enzyme inactive. Such specific expression requirements for antigens are common and this limits the types of antigens which can be used with the most common detectable marker HRP to those antigens which have no such expression requirements. In addition the fusion of an antigen and a marker at the genetic level can lead to unwanted structural/chemical alterations of the antigen or marker which in turn can lead to an imperfect detection reagent either with altered antibody binding properties or marker activity.

Seeing the problems associated with prior art methods and materials in diagnosing early the disease causing agent of febrile disease the inventors have developed a new set of materials and method for this purpose.

In accordance with a first aspect of the present invention, there is provided a method for the detection of antibodies against at least one pathogenic micro organism in a human or animal host, **characterized in that** it comprises:

a). contacting a sample from said host with a solid support coated on at least one side with an Immunoglobulin (Ig) binding protein which is directed against a specific class of Ig molecules of the species of said host, and
b). incubating the immunocomplex formed in step a). with a detection reagent comprising a complex between at least two and preferably at least four copies of an antigen from said at least one pathogenic micro organism each of which comprises a first multimerization domain and a detectable marker which comprises a second multimerization domain, wherein said complex is formed via the interaction of said first and said second multimerization domains,
c). determining the presence of said detection reagent in association with said immunocomplex formed in step a).

The Inventors have found that using a multivalent detection reagent and in particular a tetravalent detection reagent, the problems of specificity and efficiency of detection are overcome. The inventors have used again the ED3 domain from Flavivirus to validate this new method. The inventors have also found that using a detection reagent which consists of a multivalent antigen complexed with a marker, difficult to express antigens can be combined with markers that can not be expressed in the same peptide with a marker.

In the present Patent Application a complex between the antigenic peptide and a marker is a molecular structure in which the components therein are linked by non-covalent bonds.

In the present Patent Application a multivalent detection reagent consists of at least two antigens and preferably at least four antigens.

In the present Patent Application an antigen is a substance that prompts the generation of antibodies and can cause an immune response. In particular an antigen may be in the form of a peptide this being an antigenic peptide. Alternatively an antigen may be a lipid, saccharide or other molecule and/or a combination of any of these for instance a glycolipid or glycoprotein.

In the present Patent Application a copy of an antigen may comprise a number of epitopes each of which may be targeted by one or more antibodies.

In the present Patent Application the antigen can each comprise a first multimerization or alternatively groups of for instance two or more antigens may comprise a first multimerization domain.

In particular the Ig binding protein is selected in the group consisting of anti-IgM, anti-IgG and anti-IgA antibodies.

In particular the antigen is from a pathogenic micro organism which causes a febrile illness and in particular is from an organism selected from the group consisting of: a pathogenic virus, selected from the family *Adenoviruses, Herpesviruses, Poxviruses, Parvoviruses, Reoviruses, Picornaviruses, Togaviruses, Orthomyxoviruses, Rhabdoviruses, Retroviruses, Hepadnaviruses;* a pathogenic bacteria, selected from the Genus *Bordetella, Borrelia, Brucella, Campylobacter, Chlamydia, Clostridium, Corynebacterium, Enterococcus, Escherichia, Francisella, Haemophilus, Helicobacter, Legionella, Listeria, Mycobacterium, Mycoplasma, Neisseria, Pseudomonas, Rickettsia, Salmonella, Shigella, Staphylococcus, Streptococcus, Treponema, Vibrio, Yersinia;* a pathogenic protozoa from the phylum *Apicomplexa, Sarcomastigophora, Microspora, Ciliophora;* in particular said antigen is the ED3 domain polypeptide of a flavivirus and is selected from the group consisting of: a yellow fever virus ED3 domain polypeptide, a West Nile virus ED3 domain polypeptide, a Dengue virus ED3 domain polypeptide, a St Louis encephalitis virus ED3 domain polypeptide, a Murray Valley encephalitis virus ED3 domain polypeptide and a Japanese encephalitis virus ED3 domain polypeptide.

This new method using multivalent antigen detection reagents can be used to detect a number of pathogenic micro organisms of the various types listed above.

In particular the detectable marker is selected from the group consisting of: an enzyme which catalyses the production of a detectable product, a fluorescent, radioactive or metal detectable tag, in particular wherein said detectable marker is selected from the group horseradish peroxidase (HRP), alkaline phosphatase (PhoA), a fluorophore or colloidal gold.

In particular the detection reagent also comprises a polypeptide tag selected in the group consisting of HIS, c-MYC, HA, VSV-G, HSV, V5 and FLAG.

These polypeptide tags can be used to identify the detection reagent *in situ,* for instance as an antibody mediated detection marker or as a site to attach a fluorophore and/or can be used to affinity purify the detection reagent.

In particular the method comprises detecting antibodies against a panel of detection reagents each comprising a different antigen from the same pathogenic micro organism or a group of different pathogenic micro organisms, wherein the immunocomplex formed in step a). are incubated with said panel of detection reagents and the presence of said panel of detection reagents in association with said immunocomplex formed in step a). is detected.

The present method therefore can be used to detect either simultaneously using a panel of reagents comprising antigens from different pathogens upon a panel of bound serum antibodies or alternatively sequentially upon the same bound serum antibody sample the presence or absence of several pathogens and/or antigens from the same pathogen.

In particular the method may also comprise the formation of two sets of immunocomplexes as formed in step a)., a first immunocomplex consisting of a first solid surface coated in IgG binding proteins bound to IgG molecules in said sample and a second immunocomplex consisting of a second solid surface coated in IgM binding proteins bound to IgM molecules in said sample; following which;

said first and said second immunocomplexes are incubated with a detection reagent comprising a at least two and preferably at least four copies of an antigen from said at least one pathogenic micro organism each of which comprises a first multimerization domain and a detectable marker which comprises a second multimerization domain, wherein said complex is formed via the interaction of said first and said second multimerization domains, and

the amount of said detection reagent in association with said first and said second immunocomplexes is determined quantitatively and compared.

This comparison of the levels of reacting IgM and IgG antibodies allows an investigator to determine whether an infection is a primary or secondary infection with the pathogen, as in a primary infection the level of IgM would be expected to be much elevated in comparison to IgG whereas for secondary (and subsequent) infections the levels of IgM/IgG would be comparable or show more IgG than IgM.

In accordance with a second aspect of the present invention there is provided a detection reagent, comprising:

> an antigenic peptide comprising at least one antigen from a pathogenic micro organism and a first multimerization domain;
> a detectable marker and a second multimerization domain;
> wherein said detection reagent comprises at least two and preferably at least four antigenic peptides in complex with said detectable marker via the interaction of said first and said second multimerization domains.

In the present Patent Application the first and second multimerization domains may be any peptide, saccharide or other chemical moieties which cause a first molecule comprising the first multimerization domain to form a complex with a second molecule comprising the second multimerization domain. In particular the first and second multimerization domains are naturally occurring or adapted systems which involve the recognition and binding of one molecule to another such as the recognition and binding of avidin to biotin or any of the various known variants of this system. Alternatively the first and second multimerization domains may be an epitope and an antibody or a fragment thereof which recognises and binds to the epitope. The multimerization domains may also be protein domains which *in vivo* cause protein-protein multimerization such as tubulin dimerisation domains, actin recognition and binding domains or any other protein-protein binding domain. The first and second multimerization domains can also consist of reactive chemical groups such as thiol groups, maleimide groups or other suitable bond forming chemical groups. The function of the first and second multimerization domains is therefore to create a stable molecular complex between several copies of an antigenic peptide and a detectable marker or alternatively, several different antigenic peptides and a detectable marker.

In particular the first and second multimerization domains could be a coiled coil domain (Blondel & Bedouelle, 1991; Müller et al., 2000); a four helix-bundle (Pack & Plückthun, 1992); the pentamerization domain of the *E. coli* verotoxin (Zhang et al., 2004).

In another embodiment of the present invention the antigenic peptide may comprise a single polypeptide chain, comprising several copies of the same antigenic peptide, such an arrangement could be constructed at the genetic level using known molecular biology techniques.

This new class of reagent shows vastly improved performance over existing reagents for use in ELISA and in particular MAC-ELISA assays. In addition the fact that the antigenic peptide is in complex with a separate detectable marker allows the combination of antigens with markers that were not previously possible. For instance certain antigens comprise disulfide bonds or other complex chemical structures which require specific conditions to form. ED3 is an example of such an antigen. To produce this antigen or an antigenic peptide comprising this antigen or a fragment thereof, it is necessary either for the protein to be exported into the periplasmic space or alternatively produced as inclusion bodies in the cytoplasm and refolded *in vitro* so as to provide the required conditions for the formation of the disulfide bond. It is therefore difficult to produce a unified construct comprising the antigenic and marker functional portions. Further it is difficult to generate a multivalent antigen construct wherein each of the antigens is presented correctly so that they can interact with bound serum antibodies. The inventors have overcome these problems by generating two separate components, an antigenic peptide and a detectable marker each of which comprises a multimerization domain and via this multimerization domain several copies of the antigenic protein form a molecular complex with the detectable marker. This separation of the antigenic and detectable portions of the final detection reagent and the defined means by which the two components can polymerise with each other ensures that the important structure of the antigen is maintained in the final detection reagent and also the relative quantities of antigenic peptide:detectable marker can be adjusted by modifying the multimerization domains used.

In the present Patent Application by molecular complex it is intended to mean a non-covalent molecular assembly between the antigenic peptide and detectable marker.

Detection reagents consisting of antigenic peptides covalently attached to the detectable marker are also encompassed by the present invention.

In addition to the use of this reagent in MAC-ELISA it can also be used as an adsorbed monovalent antigen in an IgG-specific indirect ELISA.

In particular the antigenic peptide and/or detectable marker may comprise an affinity tag.

In particular the antigenic peptide comprises a hexahistidine affinity tag, a flaviviral ED3 domain, selected from the group consisting of: a yellow fever virus ED3 domain polypeptide, a West Nile virus ED3 domain polypeptide, a Dengue virus ED3 domain polypeptide, a St Louis encephalitis virus ED3 domain polypeptide, a Murray Valley encephalitis virus ED3 domain polypeptide and a Japanese encephalitis virus ED3 domain polypeptide and wherein the first multimerization domain comprises a biotinylation site or avidin binding peptide for instance Strep-tag™ (Schmidt et al 2007); and

said detectable marker comprises a Horseradish Peroxidase enzyme and a second multimerization domain which is selected from the group consisting of avidin, strepavidin, neutravidin or a functional derivative thereof.

The inventors have therefore extended the concept of simplified MAC-ELISA to include the use of HRP. As explained above the extension of the inventors prior work to include HRP could not be implemented simply by creating hybrid proteins between the ED3 domain and HRP at the genetic level because HRP is a monomeric and cytoplasmic protein whereas ED3 carries an essential disulfide bond that cannot form in the reducing environment of the cytoplasm. To solve this problem, the Inventors constructed recombinant plasmids that directed the expression of bt-ED3-H6 hybrids between (i) an AviTag peptide, which is biotinylated *in vivo* and that is labelled "bt" in this Patent Application; (ii) the ED3 domain from the flavivirus under consideration; and (iii) an hexahistidine for its immobilisation on a nickel ion column and purification by affinity chromatography. These hybrids were produced in the cytoplasm of *E. coli,* refolded *in vitro,* purified and then associated with a $(SA)_4$-HRP conjugate between the tetramer of streptavidin (SA) from *Streptomyces avidinii* and HRP.

The (bt-ED3-H6:$SA)_4$-HRP complexes displayed the ED3 domain in a form that was tetravalent and strongly associated with HRP. The complex corresponding to serotype 1 of dengue virus was produced and evaluated with success in a MAC-ELISA of serums from human patients that had been infected by one of the four serotypes of DENV.

HRP is a preferred marker as many substrates exist for HRP, giving chromogenic, fluorescent, or luminescent products. All of these could be used to generate a measurable signal in a MAC-ELISA performed with the (bt-ED3-H6:$SA)_4$-HRP complexes.

The present invention also relates to multivalent antigen complexes linked via streptavidin or via other means, with other enzymes (e.g. alkaline phosphatases, luciferases), fluorescent proteins, fluorophores, radioactive tracers, etc, and used in all the applications where multivalency is necessary for an assay or brings additional sensitivity, whether *in vivo, ex vivo* or *in vitro.*

In particular the peptide sequence of the antigenic peptide is selected from the group consisting of: SEQ ID NO: 1; SEQ ID NO: 4; SEQ ID NO: 5; SEQ ID NO: 6; SEQ ID NO: 7; SEQ ID NO: 8; SEQ ID NO: 9; SEQ ID NO: 10.

According to another aspect of the present invention there is provided a nucleic acid encoding the antigenic peptide or detectable marker according the second aspect of the present invention.

According to a further aspect of the present invention there is provided a method of preparing a detection reagent according to the present invention, wherein it comprises the steps:

a). preparing an expression vector containing a sequence encoding said antigenic peptide according to the present invention;

b). transforming an appropriate *E. coli* strain, with said expression vector and culturing said transformed strains;

c). purifying said antigenic peptide from the periplasmic extract by affinity purification for said affinity tag;

d). mixing said purified antigenic peptide with said detectable marker under conditions which permit the formation of a complex between said purified antigenic peptide and said detectable marker via said first and said second multimerization domains.

The preparation of recombinant antigens, produced in *E. coli,* offers many advantages over the more traditional and empirical methods, e.g. the preparation of brain extracts from suckling mice or of supernatants from cell cultures infected with the virus. The antigenic peptide component of detection reagents according to the present invention can be produced in *E. coli* and purified with low levels of security and expertise, in contrast to the more traditional preparations of antigens, which must be performed in high level security laboratories.

In addition wherein the antigenic peptide comprises a biotinylation site, biotinylation of this site can be performed *in vivo* and this is considered preferable to enzymatic biotinylation *in vitro,* which involves additional method steps. The inventors also consider in vivo biotinylation preferable to an *in vitro* chemical biotinylation, which generally occurs at random sites of the target polypeptides, e.g. the amine groups, results in an inhomogeneous population of conjugated molecules and may mask the epitopes of the antigenic peptide.

According to a further aspect of the present invention there is provided a kit for diagnosing and/or screening for anti-flavivirus antibodies in a subject comprising:

- a solid support sensitized with an Ig binding protein which is directed against a specific class of Ig molecules of the animal species under consideration,
- a detection reagent as according to the present invention,
- at least one positive control, preferably a reference serum from an infected individual and
- at least one negative control, preferably a reference serum from a non-infected individual.

In particular the kit comprises an Ig binding protein selected from the group consisting of anti-IgM, anti-IgG and anti-IgA, and said detection reagent comprises a hexahistidine tag, a flavivirus ED3 domain and the Horseradish Peroxidase enzyme.

According to a further aspect of the present invention there is provided a method for the detection of antibodies against at least one pathogenic micro organism in a human or animal host, **characterized in that** it comprises:

a). contacting a sample from said host with a solid support coated on at least one side with an Immunoglobulin (Ig) binding protein which is directed against a specific class of Ig molecules of the species of said host, and
b). incubating the immunocomplex formed in step a). with a detection reagent comprising a complex between at least four copies of an antigen from said at least one pathogenic micro organism and a detectable marker,
c). determining the presence of said detection reagent in association with said immunocomplex formed in step a).

For a better understanding of the invention and to show how the same may be carried into effect, there will now be shown by way of example only, specific embodiments, methods and processes according to the present invention with reference to the accompanying drawings in which:

**Figure 1.** Simplified MAC-ELISA of human serums, performed with the $(bt\text{-}ED3.DEN1\text{-}H6{:}SA)_4$-HRP complex. The reaction of HRP with the TMB substrate system was run for 20 min and stopped with sulphuric acid. The relative concentration of complex (starting preparation at approximately 3.15 μM, see Materials & Methods) is given along the *x*-axis and the measured signal ($A_{450nm}/A_{620nm}$) along the *y*-axis. The graph gives the average value and standard error in three independent experiments. The error bar is within the width of symbol for some data points. •, DENV1 serum N° 1; ■, DENV1 serum N° 2; ♦, DENV2 serum; ▲, DENV3 serum; ▼, DENV4 serum; ○, control serum; □, blank sample (no serum, no complex).

**Figure 2.** Dose-response relation of a MAC-ELISA performed with the $(bt\text{-}ED3\text{-}H6{:}SA)_4$-HRP complex. The complex was used at a 1280-fold dilution and the reaction of HRP with the TMB substrate system was run for 20 min. The relative concentration of serum is given along the *x*-axis and $A_{620nm}$ along the *y*-axis, after subtraction of the blank (no serum, $A_{620nm}$ = 0.1). The continuous line corresponds to the fitting of a saturation function to the experimental data. The $[Serum]_{1/2}$ value was equal to 3.7 ± 0.3 ‰ and the end-point of dilution to 0.5 ‰ for the DENV1 N° 1 serum. ●, DENV1 serum N° 1; ○, control serum. Identical results but slightly more dispersed data were obtained after stopping the reaction with sulphuric acid and measuring $A_{450nm}/A_{620nm}$.

**Figure 3.** Comparison of MAC-ELISAs, performed with the bt-ED3.DEN1-H6 hybrid and the $(SA)_4$-HRP conjugate in either one or two steps. In the one step assay, the $(bt\text{-}ED3\text{-}H6{:}SA)_4$ complex was preformed whereas in the two step assay, bt-ED3-H6 was added first, and then $(SA)_4$-HRP was added subsequently (see Materials & Methods). The reaction of HRP with the TMB substrate system was run for 20 min and stopped with sulphuric acid. The relative concentrations of the various complexes are given along the *x*-axis and the measured signal ($A_{450nm}/A_{620nm}$) along the *y*-axis. ●, DENV1 serum N° 1, one-step assay; ■, negative serum, one step ; ♦, blank sample (no serum), one step; ○, DENV1 serum N° 1, two-step assay; □, negative serum, two-steps; ◊, blank sample, two-steps.

**Figure 4.** Dose-response relation of an IgG-specific indirect ELISA performed with adsorbed bt-ED3-H6 hybrid. The reaction of HRP with the TMB substrate system was run for 15 min. The relative concentration of serum is given along the *x*-axis and $A_{620nm}$ along the *y*-axis, after subtraction of the blank (no serum, $A_{620nm}$ = 0.07). The continuous lines correspond to the fitting of a saturation function to the experimental data. The $[Serum]_{1/2}$ values were equal to 0.16 ± 0.02 ‰ and 0.22 ± 0.02 ‰ for the DENV1 serums N° 1 and N° 2 respectively, and the end-point of dilution to 0.01 ‰ for both serums. □, DENV1 serum N° 1; ■, DENV1 serum N° 2; ○, control serum. Identical results but slightly more dispersed data were obtained after stopping the reaction with sulphuric acid and measuring $A_{450nm}$/ $A_{620nm}$.

There will now be described by way of example a specific mode contemplated by the Inventors. In the following description numerous specific details are set forth in order to provide a thorough understanding. It will be apparent however, to one skilled in the art, that the present invention may be practiced without limitation to these specific details. In other instances, well known methods and structures have not been described so as not to unnecessarily obscure the description.

**Example 1. Materials and methods**

**Abbreviations**

gpE, envelope glycoprotein E; sE, extracellular domain of gpE; ED3, domain 3 of gpE; ED3.DEN1, ED3 from serotype DENV1 of dengue virus; ELISA, enzyme-linked immunosorbent assay; MAC-ELISA, IgM antibody specific capture ELISA; VLP, virus like particle; RT-PCR, a combination of reverse transcription and polymerase chain reactions; bt, biotinylated AviTag peptide; SA, streptavidin monomer; $(SA)_4$, SA tetramer; HRP, horse radish peroxidase.

**Media, buffers and kits**

The culture media LB and 2-YT (Invitrogen) have been described (Sambrook and Russell 2001). Ampicillin was used at 200 μg/mL, kanamycin at 50 μg/mL, tetracycline at 15 μg/mL and chloramphenicol at 10 μg/mL. LB medium with ampicillin was used for all the genetic constructions. PBS (phosphate buffered saline), Tween 20, the streptavidin-alkaline phosphatase conjugate, the streptavidin-horseradish peroxidase conjugate, the (anti-human IgG)-peroxidase conjugate, oxidized L-glutathione, reduced L-glutathione and The 3,3',5,5'-tetramethylbenzidine (TMB) liquid substrate system were purchased from Sigma-Aldrich; bovine serum albumin (BSA) from Roche; low-fat dry milk from Regilait. Buffer A contained 20 mM Tris-HCl, pH 8.0, 500 mM NaCl; buffer B, 100 mM $NaH_2PO_4$, 10 mM Tris-HCl, 8 M urea, 10 mM 2-mercaptoethanol, pH 8.0; buffer C, 20 mM Tris-HCl, pH 8.0, 100 mM NaCl; buffer D, 1 mM reduced L-glutathione, 0.5 mM oxidized L-glutathione in buffer C; buffer E, 0.1 % Tween 20 in PBS; buffer F, 1 % low-fat dried milk in buffer E; buffer G, 0.5 % Tween 20 and 5 % low-fat dried milk in PBS.

**Bacterial, plasmid and viral strains**

The *E. coli* strains XL1-Blue (Bullock et al. 1987), NEB-Express ($F^-$, *lon, ompT*; New England Biolabs) and AVB99 (pBirAcm in XL1-Blue; Avidity); and plasmids pET20b+ (Novagen), pLB11 (Lisova et al. 2007) and pAT224 (GenBank accession no. AY327139) (Binz et al. 2004) have been described. Plasmid pBirAcm carries the *birA* gene under control of the *ptac* promoter, and the *lacI^q* gene, both from *E. coli.* pLB11 is a derivative of pET20b+ and codes for a hybrid ED3.DEN1-H6 between the ED3 domain (residues 296-400) of DENV1 (strain FGA/89, GenBank accession N° AF226687) and a hexahistidine. pAT224 codes for a hybrid AviTag-MalE-H6 between the AviTag peptide, the MalE protein of *E. coli* and a hexahistidine, under control of the T5 promoter and *lacO* operator; it also carries the *lacI^q* gene. Strain NZ2 was constructed by introducing the pBirAcm plasmid of AVB99 into NEB-Express by transformation.

**Antibodies and antiserums**

Human sera were from the collection of the National Reference Center (NRC) for Arboviruses, Institut Pasteur de la Guyane, French Guiana. They were collected from patients who displayed clinical symptoms of dengue (temperature of ≥ 38.5 °C, headache, myalgia and/or arthralgia), associated or not with rash and minor hemorrhagic manifestations, in the context of the NRC activity. Six serum samples were tested: two from patients infected with DENV1, one with DENV2, one with DENV3, one with DENV4, and one from an individual who was not infected by DENV. The samples were collected during the early convalescent phase of the disease, i. e. 5-6 days or later after fever onset. They were characterized by standard diagnostic methods, i. e. MAC-ELISA and antigen capture IgG indirect ELISA, using mouse-brain extracts as antigens (Dussart et al. 2008). The DENV serotype that was responsible for the disease, was identified by RT-PCR as described (Lanciotti et al. 1992). This identification was performed on serum samples that were collected from the patients in the acute phase of the disease. Microtiter plates coated with antibodies directed against human immunoglobulin μ-chains (Platelia™ IgM microplates) were purchased from Bio-Rad.

**Construction of the *AviTag-ED3-H6* hybrid gene**

The *AviTag-ED3.DEN1-H6* hybrid gene (SEQ ID NO: 1), coding for a hybrid protein between the AviTag, the ED3.DEN1 domain and a hexahistidine, was constructed as follows. Plasmids pLB11 and pAT224 were digested with the restriction enzymes NcoI and BlpI. The products of digestion were purified with a PCR purification kit (Qiagen) to remove the BlpI enzyme, which is heat stable, ligated and then introduced into XL1-Blue by transformation. The recombinant plasmids were identified by digestion with the restriction enzyme NdeI and the sequence of the hybrid gene checked by DNA sequencing. The resulting plasmid, pNZ21, coded for the AviTag-ED3.DEN1-H6 hybrid protein (SEQ ID NO: 2), under control of the T5 promoter and *lacO* operator.

**Production and purification of the bt-ED3-H6 hybrid**

The bt-ED3-H6 hybrid was expressed from plasmid pNZ21 in strain NZ2. A pre-culture of the producing strain was obtained by inoculation of 2xYT broth, containing ampicillin and chloramphenicol, with an isolated colony and overnight incubation at 30 °C. The production was obtained by dilution of the pre-culture in one volume of 2xYT broth, containing ampicillin, to obtain an initial absorbance $A_{600nm}$ = 0.1, growth at 30 °C until $A_{600nm}$ = 1.0, induction of the *ptac* and T5 promoters with 1.0 mM IPTG, addition of 50 mM biotin, and further incubation for 4 h at the same temperature. The subsequent steps were performed at 4 °C. The culture was cooled on ice and then centrifuged 10 min at 5000 rpm to pellet the bacteria. The bacterial pellet was resuspended in 1/20th volume of 1 mg/mL hen egg white lysozyme, 5 mM imidazole in buffer A and the cells were broken open by sonication. The insoluble materials were pelleted by centrifugation for 20 min at 10,000g and the supernatant discarded.

The subsequent steps were performed at room temperature to avoid crystallisation of urea. The insolubles were resuspended in 1/20th volume of buffer B. The suspension was mildly agitated until it became translucent, and then centrifuged 20 min at 10,000g to remove the cell debris. The supernatant contained the solubilized proteins. The bt-ED3-H6 hybrid was separated from the other solubilized proteins by affinity chromatography on a column of NiNTA resin (2-3 mL/L of culture, Qiagen), and refolded in the column before elution. The column was equilibrated with 6 volumes of buffer B, loaded with the preparation of solubilized proteins, and then washed with 20 volumes of buffer C to remove urea and the unbound proteins.

The subsequent steps were performed at 4 °C. The column was equilibrated with 5 volumes of buffer D, its outlet was capped, 1 volume of buffer D was added and the reaction of refolding was left to run overnight. The column was washed with 5 volumes of buffer A to remove glutathione and then with 5 mM imidazole in buffer A to remove the weakly bound proteins. bt-ED3-H6 was then eluted by a step gradient of imidazole in buffer A. The fractions of purifications were analyzed by SDS-PAGE (12% acrylamide, NuPage Novex system, Invitrogen) in reducing conditions. The purest fractions were pooled, snap-frozen and kept at -80 °C. They were homogeneous at > 98 %.

**Proteins and their complexes**

The molecular mass MM and extinction coefficient $\varepsilon_{280nm}$ of the proteins were calculated from their amino acid sequences with the subroutine Pepstats of the software suite EMBOSS (Rice et al. 2000). The MM value of the biotin moiety is equal to 226 (Beckett et al. 1999). The MM value of the bt-ED3-H6 hybrid was equal to 14989 and its $\varepsilon_{280nm}$ value to 15220 $M^{-1}$ $cm^{-1}$. The MM value of the strepta-vidine monomer from *Streptomyces avidinii* (GenBank accession N° CAA00084) was equal to 16622, that of horse radish peroxidase to 44000 (Sigma-Aldrich) and therefore that of the $(SA)_4$-HRP conjugate to 110488 (Sigma-Aldrich). The (bt-ED3-H6:SA)$_4$-HRP complex was prepared by mixing the bt-ED3-H6 hybrid and $(SA)_4$-HRP conjugate in a 12:1 molar ratio (i.e. 3 moles of bt-ED3-H6 for 1 mole of SA monomer; see Results). More precisely, a preparation of complex was formed extemporaneously by mixing 13.1 μL of bt-ED3.DEN1-H6 hybrid (purified preparation at 0.87 mg/mL) and 7 μL of $(SA)_4$-HRP conjugate (stock solution at 1 mg/mL in PBS), followed by incubation for 1 hour at room temperature. The concentration of complex was therefore equal to 3.15 μM in the resulting preparation. Two additional mixes were prepared for control experiments. Mix-1 was prepared by mixing 13.1 μL of the purified preparation of bt-ED3.DEN1-H6 and 7 μL PBS; Mix-2, by mixing 13.1 μL of PBS and 7 μL of the stock solution of $(SA)_4$-HRP.

**MAC-ELISA**

The IgM antibody specific capture ELISA (MAC-ELISA) was performed using microtiter plates coated with anti-human IgM and a volume of 100 μL/well. The human serum samples were diluted 100-fold in buffer F, unless otherwise indicated. The preparation of (bt-ED3-H6:SA)$_4$-HRP complex was diluted 10- to 1280-fold in buffer F. Wells from one column of the microplate were loaded with the diluted serums or buffer F for the blank. The plate was incubated for 1 h at 37 °C for the reaction of antibody capture. The wells were washed three times with buffer E and then loaded with the dilutions of complex, except for the blank well. The plate was incubated for 1 h at 37 °C for the binding reaction. The wells were washed three times with buffer E and the bound (bt-ED3-H6:SA)$_4$-HRP molecules detected by addition of TMB. $A_{620nm}$ was measured after 5, 10, 15 and 20 min at 25 °C. The reaction was stopped by addition of 50 μL of 1 N sulfuric acid and the ratio $A_{450nm}/A_{620nm}$ measured. The signal of the serum was considered as significant if its value was at least twice that of the blank controls. This condition was used to define the end-point value of the serum concentration when the latter was varied.

In a control experiments, the dilutions of the (bt-ED3.DEN1-H6:SA)$_4$-HRP complex were replaced by those of Mix-1, containing only the bt-ED3.DEN1-H6 hybrid (see previous paragraph). The plate was incubated for 1 h at 37 °C for the binding reaction. The wells were washed three times with buffer E and then loaded with the corresponding dilutions of Mix-2, containing only the (SA)$_4$-HRP conjugate. The plate was incubated for 1 h at 37 °C for the capture of the conjugate by the bound bt-ED3.DEN1-H6 hybrid. The wells were washed three times with buffer E and then the reaction with TMB was run as above.

**IgG-specific indirect ELISA**

The indirect ELISAs were performed in microtiter plates (Polysorb, Nunc) with volumes of 100 μL/well. The wells of a plate were sensitized with the bt-ED3.DEN1-H6 hybrid. The hybrid (0.3 μg/mL in PBS) was loaded in the wells and the plate incubated overnight at 4 °C. The wells were washed three times with buffer E and then blocked with buffer G for 1 h at 37 °C. The wells were washed as above. The positive and negative human serums were diluted 400- to 102400-fold in buffer F and then loaded in duplicate in the sample wells while buffer F alone was loaded in the blank wells. The plate was incubated for 1 h at 37 °C for the binding reaction to occur and then the wells were washed three times with buffer E. A dilution of (anti-human IgG)-HRP conjugate (1000-fold in buffer F) was added in the sample and blank wells. The plate was incubated for 1 h at 37 °C for the reaction of capture and then the wells were washed as above. The bound molecules of (anti-human Ig)-HRP were detected by addition of TMB. $A_{620nm}$ was measured after 5, 10 and 15 min at 25°C. The reaction was stopped by addition of 50 μL of 1 N sulfuric acid and $A_{450nm}/A_{620nm}$ measured. The signal of the serum was considered as significant if its value was at least twice that of the blank control. This condition was used to define the end-point value of the serum concentration.

**Example 2. Results**

**Construction of the biotinylated hybrids bt-ED3-H6**

The inventors constructed plasmid vectors that coded for the cytoplasmic expression of AviTag-ED3-H6 hybrids in *E. coli,* under control of the T5 promoter and *lacO* operator (Table 1).

**Table 1.** Recombinant plasmids for the expression of bt-ED3-H6 hybrids in *E. coli.* Column 2 gives the residues of the envelope protein from the virus in column 3 that are carried by the plasmid in column 1. Some codons may be synonymous but not identical in the plasmids and in the GenBank sequences. DENV1-4, serotypes 1 to 4 of dengue virus; YFV, yellow fever virus; WNV, West-Nile virus; JEV, Japanese encephalitis virus; SLEV, Saint-Louis encephalitis virus. The codons in the plasmids of column 1 might differ from those in the genes of column 6 but the amino acid sequences, restricted to the segment of column 2, were identical.

| Plasmid | Residues | Virus | Strain | Origin | GenBank No | Antigenic Peptide (SEQ ID NO) |
|---|---|---|---|---|---|---|
| pNZ21 | 296-400 | DENV1 | FGA/89 | French-Guiana | AF226687 | 1 |
| pNZ22 | 296-400 | DENV2 | Jamaica/N. 1409 | Jamaica | M20558 | 4 |
| pNZ23 | 294-398 | DENV3 | PaH881/88 | Thailand | AF349753 | 5 |
| pNZ24 | 296-400 | DENV4 | ThD4-0113-76 | Burma/ Thailand | AY618949 | 6 |
| pNZ25 | 299-406 | WNV | IS98-ST1 | Israel | AF481864 | 7 |

(continued)

| Plasmid | Residues | Virus | Strain | Origin | GenBank No | Antigenic Peptide (SEQ ID NO) |
|---|---|---|---|---|---|---|
| pNZ26 | 298-405 | JEV | SA14 | Sian, China | U14163 | 8 |
| pNZ27 | 294-398 | YFV | 17D | Vaccine Str. | X03700 | 9 |
| pNZ29 | 299-406 | SLEV | MSI7 | USA | AY289618 | 10 |

These hybrid proteins comprised three segments from N- to C-terminus: (i) the AviTag peptide, which is specifically biotinylated by biotin-protein ligase BirA, product of the *birA* gene from *E. coli* (Barker and Campbell 1981); (ii) the ED3 domain from the envelope protein of a flavivirus, which contains one disulfide bond; (iii) a hexahistidine for the immobilisation and purification of the hybrid on a nickel ion column. Plasmid pNZ21, which corresponded to the ED3 domain from DENV1, was introduced into the *E. coli* strain NZ2 for expression of the hybrid protein bt-ED3.DEN1-H6. Strain NZ2 harboured plasmid pBirA, which carried the *birA* gene under control of the *ptac* promoter. Both pNZ21 and pBirA carried the *lacI^q^* gene and both expressions of BirA and bt-ED3-H6 were under control of the LacI repressor.

**Production, *invitro* refolding and purification of the bt-ED3-H6 hybrid**

The producing bacteria were broken open by sonication and the biotinylated hybrid, bt-ED3-H6, was purified from the insoluble fraction. Briefly, the insoluble proteins were solubilized with 8 M urea and 2-mercaptoethanol to reduce the intermolecular disulfide-bonds. The solubilized proteins were loaded on a NiNTA column, the unbound proteins and 2-mercaptoethanol were washed out of the column, and then the bound proteins were refolded in the column in the presence of a mixture of reduced and oxidized glutathione to favour the formation of the correct intramolecular disulfide bond. Glutathione was washed out of the column and then the refolded proteins were eluted from the column by a step-gradient of imidazole. The purified bt-ED3-H6 hybrid was > 98 % pure. We obtained 7.1 mg of the purified, refolded, biotinylated bt-ED3.DEN1-H6 hybrid/L of culture in flask by this procedure. Efficient biotinylation was confirmed by ELISA and blotting with a streptavidin-alkaline phosphatase conjugate.

**Formation of the complex between bt-ED3-H6 and the $(SA)_4$-HRP conjugate**

The $(SA)_4$-HRP conjugate between streptavidin and horseradish peroxidase, contains four binding sites for biotin, one per SA monomer. It was essential that these four sites be occupied by the bt-ED3-H6 hybrid to generate a multivalency of the ED3 antigen and an avidity effect. We therefore run the binding reaction between bt-ED3-H6 and $(SA)_4$-HRP in a molar ratio of 12:1, which corresponded to three molecules of bt-ED3-H6 per SA monomer. This ratio was calculated under the assumption that each molecule of conjugate contained one molecule of HRP per SA tetramer. We tested different dilutions (10 to 1280-fold) of the complex preparation in MAC-ELISA of human serums (see below). We represent such a complex by $(bt\text{-}ED3.DEN1\text{-}H6{:}SA)_4$-HRP, where "-" stands for a covalent bond and ":" for a non-covalent interaction.

**Assay of human serums with a simplified MAC-ELISA**

We used the $(bt\text{-}ED3.DEN1\text{-}H6{:}SA)_4$-HRP complex, corresponding to serotype DENV1 of the dengue virus, to detect IgMs, directed against DENV1, in the serum of patients who had been infected recently. We used microtiter-plates in the wells of which an antibody, directed against human IgMs, had been immobilized. This immobilized antibody was used to capture the IgMs of the human serum. The IgMs that were directed against the ED3.DEN1 domain, were detected with $(bt\text{-}ED3.DEN1\text{-}H6{:}SA)_4$-HRP, through the binding of its ED3.DEN1 moiety and the enzymatic activity of its HRP moiety. We also tested the serums of patients who had been infected recently with the DENV2, -3 or -4 serotypes of the dengue virus, the serum of a patient who had not been infected with any DENV, and a blank sample in which both serum and complex were omitted (Figure 1). All the serums had been characterized by RT-PCR and a standard MAC-ELISA of samples that had been collected early after the onset of symptoms.

We observed an important cross-reaction of the $(bt\text{-}ED3.DEN1\text{-}H6{:}SA)_4$-HRP complex with all the samples, when it was diluted 10 to 160-fold. This cross-reaction totally disappeared for dilutions above 500-fold. A reaction time of 20 minutes was sufficient to develop the signal. At high dilution, the complex reacted very strongly with a first DENV1 serum and significantly with a second one. It reacted very weakly with the DENV2, -3 and -4 serums and with the negative serum (Figure 1). At the highest tested dilution of the complex, the average signals for the DENV1 serums were 15.6-fold and 4.2-fold higher than the signal for the uninfected serum in three independent experiments. In contrast, the signals for the other DENV serums were identical to the signal for the uninfected serum. Thus, we observed a sensitive and specific detection of DENV1 relative to the other serotypes of DENV in a simple and fast MAC-ELISA assay, as described here.

We checked that the MAC-ELISA signal was an increasing and saturating function of the concentration in DENV1-infected serum, and a constant and nil function of the concentration in non-infected serum, when the (bt-ED3.DEN1-H6:SA)$_4$-HRP complex was used at a high dilution (1280-fold; Figure 2). The dilution of the DENV1 serum N° 1 for half-maximal signal was equal to 275-fold and its end point value to 2000-fold.

**Monovalent versus multivalent presentation of the antigen**

In the MAC-ELISA of the previous paragraph, the (bt-ED3.DEN1-H6:SA)$_4$-HRP complex was pre-formed, it displayed the ED3 domain tetravalently, and its two components were captured in one step by the IgMs of the serum under assay. We also performed the capture of these two components in two steps to test the importance of the multivalency of the ED3 domain. In a first step, the bt-ED3.DEN1-H6 hybrid was presented in a free monovalent state to the IgMs that were bound to the plates. The unbound bt-ED3.DEN1-H6 molecules were subsequently washed off. In a second step, the (SA)$_4$-HRP conjugate was added for capture by the bound bt-ED3.DEN1-H6 hybrid. We found that the measured signal was similar for the infected serum, the non-infected serum and the blank control when the MAC-ELISA was performed in two steps, whereas it was 20-fold higher for the infected serum when the assay was done in one steps (Figure 3). Thus, the multivalent display of the antigen was absolutely necessary for its recognition and binding by the IgMs of the serum.

**Use of a bt-ED3-H6 hybrid in an IgG specific indirect ELISA**

The ED3 domains can be used in an isolated format to detect IgG antibodies that are directed against the cognate flaviviruses in the serum of patients (see Introduction). In such assays, the ED3 domain is immobilized in the wells of a microtiter plate by passive adsorption and captures IgG antibodies in the serum under assay. The captured IgGs are then detected with a conjugate between a secondary antibody, directed against human IgGs, and an enzyme, usually HRP. We tested whether the bt-ED3-H6 hybrids could be used in such an indirect ELISA (Figure 4). The results showed that the bt-ED3.DEN1-H6 hybrid detected anti-DENV1 IgGs in patient serums with speed (detection in 15 min), sensitivity and specificity since the serum of a non-infected patient gave a negative signal. The dilutions of the DENV1 serums N° 1 and N° 2 for half-maximal signal were equal to 6308-fold and 4530-fold respectively, and the end-points of dilution to 102400-fold for both. Thus, the same preparation of bt-ED3-H6 hybrid could be used both as a tetravalent (bt-ED3-H6:SA)$_4$-HRP complex in a MAC-ELISA and as an adsorbed monovalent antigen in an IgG-specific indirect ELISA.

**Conclusions**

The advantages of the (bt-ED3-H6:SA)$_4$-HRP complexes over the (H6-ED3-PhoA)$_2$ hybrids are important. As stated in the Introduction section, HRP is smaller, more stable and less expensive to produce than PhoA. It has a much higher catalytic turnover number that generates a strong signal in a short time span. Whereas the full-length H6-ED3-PhoA hybrid must be produced in *E. coli,* only the much smaller bt-ED3-H6 partner of the complex has to be produced in *E. coli,* the (SA)$_4$-HRP partner being commercially available at a reasonable price. Whereas the hybrid must be exported into the periplasmic space of *E. coli* for its proper folding, the bt-ED3-H6 domain can be produced in the *E. coli* cytoplasm as inclusion bodies and then folded within the purification column, according to a simple protocol. As a result, the yields of production and purification are higher for the isolated bt-ED3-H6 domain (7.1 mg/L of culture; 470 nmoles/L of culture) than for the H6-ED3-PhoA hybrid (1.0 mg monomer/L; 16 nmoles/L), especially when calculated as moles/L of culture because the MM value of the isolated domain, 14989, is much smaller than that of the hybrid, 60631. Moreover, whereas the H6-ED3-PhoA hybrid is used at a monomer concentration of 0.066 $\mu$M in MAC-ELISA, the bt-ED3-H6 domain was used at a monomer concentration of 0.0025 $\mu$M, i. e. $\geq$ 5-fold lower for a 10-fold faster generation of the chromogenic signal.

**References**

AnandaRao, R., Swaminathan, S., Fernando, S., Jana, A.M., and Khanna, N. 2005. A custom-designed recombinant multiepitope protein as a dengue diagnostic reagent. Protein Expr Purif 41: 136-147.

Barker, D.F., and Campbell, A.M. 1981. The birA gene of Escherichia coli encodes a biotin holoenzyme synthetase.

J Mol Biol 146: 451-467.
Beasley, D.W., Holbrook, M.R., Travassos Da Rosa, A.P., Coffey, L., Carrara, A.S., Phillippi-Falkenstein, K., Bohm, R.P., Jr., Ratterree, M.S., Lillibridge, K.M., Ludwig, G.V., et al. 2004. Use of a recombinant envelope protein subunit antigen for specific serological diagnosis of West Nile virus infection. J Clin Microbiol 42: 2759-2765.
Beckett, D., Kovaleva, E., and Schatz, P.J. 1999. A minimal peptide substrate in biotin holoenzyme synthetase-catalyzed biotinylation. Protein Sci 8: 921-929.
Bedouelle, H., Brient-Litzler, E., Dussart, P., Despres, P., and Bremand, L. 2008. Method for the diagnosis or the screening of an arbovirus infection, reagents useful in said method and their applications. Patent application; publication No: WO2008152528 (A2); date of priority 15/06/2007; applicants: CNRS, Institut Pasteur (DI 2007-14 and DI 2008-31).
Binz, H.K., Amstutz, P., Kohl, A., Stumpp, M.T., Briand, C., Forrer, P., Grutter, M.G., and Pluckthun, A. 2004. High-affinity binders selected from designed ankyrin repeat protein libraries. Nat Biotechnol 22: 575-582.
Blondel A, Bedouelle H. (1991). Engineering the quaternary structure of an exported protein with a leucine zipper. Protein Eng. 4:457-461. PubMed PMID: 1881871.
Bullock, W.O., Fernandez, J.M., and Short, J.M. 1987. XLI-Blue: a high efficiency plasmid transforming recA Escherichia coli strain with beta-galactosidase selection. BioTechniques 5: 376-379.
Crill, W.D., and Chang, G.J. 2004. Localization and characterization of flavivirus envelope glycoprotein cross-reactive epitopes. J Virol 78: 13975-13986.
Crill, W.D., and Roehrig, J.T. 2001. Monoclonal antibodies that bind to domain III of dengue virus E glycoprotein are the most efficient blockers of virus adsorption to Vero cells. J Virol 75: 7769-7773.
Dauphin, G., and Zientara, S. 2007. West Nile virus: recent trends in diagnosis and vaccine development. Vaccine 25: 5563-5576.
de Oliveira Poersch, C., Pavoni, D.P., Queiroz, M.H., de Borba, L., Goldenberg, S., dos Santos, C.N., and Krieger, M.A. 2005. Dengue virus infections: comparison of methods for diagnosing the acute disease. J Clin Virol 32: 272-277.
Dussart, P., Labeau, B., Lagathu, G., Louis, P., Nunes, M.R., Rodrigues, S.G., Storck-Herrmann, C., Cesaire, R., Morvan, J., Flamand, M., et al. 2006. Evaluation of an enzyme immunoassay for detection of dengue virus NS1 antigen in human serum. Clin Vaccine Immunol 13: 1185-1189.
Dussart, P., Petit, L., Labeau, B., Bremand, L., Leduc, A., Moua, D., Matheus, S., and Baril, L. 2008. Evaluation of Two New Commercial Tests for the Diagnosis of Acute Dengue Virus Infection Using NS1 Antigen Detection in Human Serum. PLoS Negl Trop Dis 2: e280.
Gould, E.A., and Solomon, T. 2008. Pathogenic flaviviruses. Lancet 371: 500-509.
Gritsun, T.S., Holmes, E.C., and Gould, E.A. 1995. Analysis of flavivirus envelope proteins reveals variable domains that reflect their antigenicity and may determine their pathogenesis. Virus Res 35: 307-321.
Halstead, S.B. 1988. Pathogenesis of dengue: challenges to molecular biology. Science 239: 476-481.
Holbrook, M.R., Shope, R.E., and Barrett, A.D. 2004. Use of recombinant E protein domain III-based enzyme-linked immunosorbent assays for differentiation of tick-borne encephalitis serocomplex flaviviruses from mosquito-borne flaviviruses. J Clin Microbiol 42: 4101-4110.
Holmes, D.A., Purdy, D.E., Chao, D.Y., Noga, A.J., and Chang, G.J. 2005. Comparative analysis of immunoglobulin M (IgM) capture enzyme-linked immunosorbent assay using virus-like particles or virus-infected mouse brain antigens to detect IgM antibody in sera from patients with evident flaviviral infections. J Clin Microbiol 43: 3227-3236.
Igarashi, A. 1997. Impact of dengue virus infection and its control. FEMS Immunol Med Microbiol 18: 291-300.
Innis, B.L., Nisalak, A., Nimmannitya, S., Kusalerdchariya, S., Chongswasdi, V., Suntayakorn, S., Puttisri, P., and Hoke, C.H. 1989. An enzyme-linked immunosorbent assay to characterize dengue infections where dengue and Japanese encephalitis co-circulate. Am J Trop Med Hyg 40: 418-427.
Johnson, A.J., Martin, D.A., Karabatsos, N., and Roehrig, J.T. 2000. Detection of anti-arboviral immunoglobulin G by using a monoclonal antibody-based capture enzyme-linked immunosorbent assay. J Clin Microbiol 38: 1827-1831.
Kanai, R., Kar, K., Anthony, K., Gould, L.H., Ledizet, M., Fikrig, E., Marasco, W.A., Koski, R.A., and Modis, Y. 2006. Crystal structure of west nile virus envelope glycoprotein reveals viral surface epitopes. J Virol 80: 11000-11008.
Kao, C.L., King, C.C., Chao, D.Y., Wu, H.L., and Chang, G.J. 2005. Laboratory diagnosis of dengue virus infection: current and future perspectives in clinical diagnosis and public health. J Microbiol Immunol Infect 38: 5-16.
Kuno, G. 2003. Serodiagnosis of flaviviral infections and vaccinations in humans. Adv Virus Res 61: 3-65.
Lanciotti, R.S., Calisher, C.H., Gubler, D.J., Chang, G.J., and Vorndam, A.V. 1992. Rapid detection and typing of dengue viruses from clinical samples by using reverse transcriptase-polymerase chain reaction. J Clin Microbiol 30: 545-551.
Lisova, O., Hardy, F., Petit, V., and Bedouelle, H. 2007. Mapping to completeness and transplantation of a group-specific, discontinuous, neutralizing epitope in the envelope protein of dengue virus. J Gen Virol 88: 2387-2397.
Ludolfs, D., Schilling, S., Altenschmidt, J., and Schmitz, H. 2002. Serological differentiation of infections with dengue virus serotypes 1 to 4 by using recombinant antigens. J Clin Microbiol 40: 4317-4320.

Makino, Y., Tadano, M., Saito, M., Maneekarn, N., Sittisombut, N., Sirisanthana, V., Poneprasert, B., and Fukunaga, T. 1994. Studies on serological cross-reaction in sequential flavivirus infections. Microbiol Immunol 38: 951-955.

Martin, D.A., Biggerstaff, B.J., Allen, B., Johnson, A.J., Lanciotti, R.S., and Roehrig, J.T. 2002. Use of immunoglobulin m cross-reactions in differential diagnosis of human flaviviral encephalitis infections in the United States. Clin Diagn Lab Immunol 9: 544-549.

Martin, D.A., Muth, D.A., Brown, T., Johnson, A.J., Karabatsos, N., and Roehrig, J.T. 2000. Standardization of immunoglobulin M capture enzyme-linked immunosorbent assays for routine diagnosis of arboviral infections. J Clin Microbiol 38: 1823-1826.

Mathew, A., and Rothman, A.L. 2008. Understanding the contribution of cellular immunity to dengue disease pathogenesis. Immunol Rev 225: 300-313.

Modis, Y., Ogata, S., Clements, D., and Harrison, S.C. 2003. A ligand-binding pocket in the dengue virus envelope glycoprotein. Proc Natl Acad Sci U S A 100: 6986-6991.

Modis, Y., Ogata, S., Clements, D., and Harrison, S.C. 2004. Structure of the dengue virus envelope protein after membrane fusion. Nature 427: 313-319.

Modis, Y., Ogata, S., Clements, D., and Harrison, S.C. 2005. Variable surface epitopes in the crystal structure of dengue virus type 3 envelope glycoprotein. J Virol 79: 1223-1231.

Mongkolsapaya, J., Dejnirattisai, W., Xu, X.N., Vasanawathana, S., Tangthawornchaikul, N., Chairunsri, A., Sawasdivom, S., Duangchinda, T., Dong, T., Rowland-Jones, S., et al. 2003. Original antigenic sin and apoptosis in the pathogenesis of dengue hemorrhagic fever. Nat Med 9: 921-927.

Mukhopadhyay, S., Kuhn, R.J., and Rossmann, M.G. 2005. A structural perspective of the flavivirus life cycle. Nat Rev Microbiol 3: 13-22.

Müller KM, Arndt KM, Alber T. (2000) Protein fusions to coiled-coil domains. Methods Enzymol. 328:261-282. PubMed PMID: 11075350.

Pack P, Plückthun A. (1992). Miniantibodies: use of amphipathic helices to produce functional, flexibly linked dimeric FV fragments with high avidity in Escherichia coli. Biochemistry. 31:1579-1584. PubMed PMID: 1737014.

Potts, J.A., and Rothman, A.L. 2008. Clinical and laboratory features that distinguish dengue from other febrile illnesses in endemic populations. Trop Med Int Health 13: 1328-1340.

Rey, F.A., Heinz, F.X., Mandl, C., Kunz, C., and Harrison, S.C. 1995. The envelope glycoprotein from tick-borne encephalitis virus at 2 A resolution. Nature 375: 291-298.

Rice, P., Longden, I., and Bleasby, A. 2000. EMBOSS: the European Molecular Biology Open Software Suite. Trends Genet 16: 276-277.

Sambrook, J., and Russell, D.W. 2001. Molecular Cloning: A Laboratory Manual, 3rd ed. Cold Spring Harbor Press, Cold Spring Harbor, New York.

Sanchez, M.D., Pierson, T.C., McAllister, D., Hanna, S.L., Puffer, B.A., Valentine, L.E., Murtadha, M.M., Hoxie, J.A., and Doms, R.W. 2005. Characterization of neutralizing antibodies to West Nile virus. Virology 336: 70-82.

Schilling, S., Ludolfs, D., Van An, L., and Schmitz, H. 2004. Laboratory diagnosis of primary and secondary dengue infection. J Clin Virol 31: 179-184.

Schwartz, E., Mileguir, F., Grossman, Z., and Mendelson, E. 2000. Evaluation of ELISA-based sero-diagnosis of dengue fever in travelers. J Clin Virol 19: 169-173.

Schmidt TGM and Skerra A, 2007. The Strep-tag system for one-step purification and high-affinity detection or capturing of proteins. NATURE PROTOCOLS 2, 1528-1535

Shu, P.Y., and Huang, J.H. 2004. Current advances in dengue diagnosis. Clin Diagn Lab Immunol 11: 642-650.

Sigma-Aldrich. Product information on peroxidase from horseradish, Catalog number P8375. *p8375dat.pdf.*

Sigma-Aldrich. Product information on Streptavidin-Peroxidase from Streptomyces avidinii, catalog number S5512. *s5512pis.pdf.*

Simmons, M., Porter, K.R., Escamilla, J., Graham, R., Watts, D.M., Eckels, K.H., and Hayes, C.G. 1998. Evaluation of recombinant dengue viral envelope B domain protein antigens for the detection of dengue complex-specific antibodies. Am J Trop Med Hyg 58: 144-151.

Takasaki, T., Nawa, M., Yamada, K.I., Harada, M., Takeda, A., and Kurane, I. 2002. Evaluation of dengue IgM detection tests using sera from patients with autoimmune diseases. J Virol Methods 102: 61-66.

Teles, F.R., Prazeres, D.M., and Lima-Filho, J.L. 2005. Trends in dengue diagnosis. Rev Med Virol 15: 287-302.

Weber, P.C., Ohlendorf, D.H., Wendoloski, J.J., and Salemme, F.R. 1989. Structural origins of high-affinity biotin binding to streptavidin. Science 243: 85-88.

Westaway, E.G., Shew, M., and Della-Porta, A.J. 1975. Reactions of purified hemagglutinating antigens of flaviviruses with 19S and 7S antibodies. Infect Immun 11: 630-634.

WHO. 1997. *Dengue Haemorrhagic Fever: diagnosis, prevention, treatment and control.,* Second ed. WHO Publications, Geneva.

Wilchek, M., Bayer, E.A., and Livnah, O. 2006. Essentials of biorecognition: the (strept)avidin-biotin system as a model for protein-protein and protein-ligand interaction. Immunol Lett 103: 27-32.

Yamada, K., Nawa, M., Takasaki, T., Yabe, S., and Kurane, I. 1999. Laboratory diagnosis of dengue virus infection by reverse transcriptase polymerase chain reaction (RT-PCR) and IgM-capture enzyme-linked immunosorbent assay (ELISA). Jpn J Infect Dis 52: 150-155.

Zhang J, Tanha J, Hirama T, Khieu NH, To R, Tong-Sevinc H, Stone E, Brisson JR, MacKenzie CR (2004). Pentamerization of single-domain antibodies from phage libraries: a novel strategy for the rapid generation of high-avidity antibody reagents. J Mol Biol. 335:49-56. PubMed PMID: 14659739.

## Claims

1. A method for the detection of antibodies against at least one pathogenic micro organism in a human or animal host, **characterized in that** it comprises:

    a). contacting a sample from said host with a solid support coated on at least one side with an Immunoglobulin (Ig) binding protein which is directed against a specific class of Ig molecules of the species of said host, and
    b). incubating the immunocomplex formed in step a). with a detection reagent comprising a complex between at least two and preferably at least four copies of an antigen from said at least one pathogenic micro organism each of which comprises a first multimerization domain and a detectable marker which comprises a second multimerization domain, wherein said complex is formed via the interaction of said first and said second multimerization domains,
    c). determining the presence of said detection reagent in association with said immunocomplex formed in step a).

2. The method of claim 1, wherein said Ig binding protein is selected in the group consisting of anti-IgM, anti-IgG and anti-IgA antibodies.

3. The method of claim 1 or claim 2, wherein said antigen is from a pathogenic micro organism which causes a febrile illness and in particular is from an organism selected from the group consisting of: a pathogenic virus, selected from the family *Adenoviruses, Herpesviruses, Poxviruses, Parvoviruses, Reoviruses, Picornaviruses, Togaviruses, Or-*

*thomyxoviruses, Rhabdoviruses, Retroviruses, Hepadnaviruses;* a pathogenic bacteria, selected from the Genus *Bordetella, Borrelia, Brucella, Campylobacter, Chlamydia, Clostridium, Corynebacterium, Enterococcus, Escherichia, Francisella, Haemophilus, Helicobacter, Legionella, Listeria, Mycobacterium, Mycoplasma, Neisseria, Pseudomonas, Rickettsia, Salmonella, Shigella, Staphylococcus, Streptococcus, Treponema, Vibrio, Yersinia;* a pathogenic protozoa, selected from the phylum *Apicomplexa, Sarcomastigophora, Microspora, Ciliophora;* in particular said antigen is the ED3 domain polypeptide of a flavivirus and is selected from the group consisting of: a yellow fever virus ED3 domain polypeptide, a West Nile virus ED3 domain polypeptide, a Dengue virus ED3 domain polypeptide, a St Louis encephalitis virus ED3 domain polypeptide, a Murray Valley encephalitis virus ED3 domain polypeptide and a Japanese encephalitis virus ED3 domain polypeptide.

**4.** The method of any one of claims 1 to 3, **characterized in that** said detectable marker is selected from the group consisting of: an enzyme which catalyses the production of a detectable product, a fluorescent, radioactive or metal detectable tag, in particular wherein said detectable marker is selected from the group horseradish peroxidase (HRP), a fluorophore or colloidal gold.

**5.** The method of any one of claims 1 to 4, wherein said detection reagent further comprises a polypeptide tag selected in the group consisting of HIS, c-MYC, HA, VSV-G, HSV, V5 and FLAG.

**6.** The method of any one of claims 1 to 5, wherein said method comprises detecting antibodies against a panel of detection reagents each comprising a different antigen from the same pathogenic micro organism or a group of different pathogenic micro organisms, wherein the immunocomplex formed in step a). is incubated with said panel of detection reagents and the presence of said panel of detection reagents in association with said immunocomplex formed in step a). is detected.

**7.** The method of any one of claims 1 to 6, wherein two sets of immunocomplexes are formed in step a)., a first immunocomplex consisting of a first solid surface coated in IgG binding proteins bound to IgG molecules in said sample and a second immunocomplex consisting of a second solid surface coated in IgM binding proteins bound to IgM molecules in said sample; following which;
said first and said second immunocomplexes are incubated with a detection reagent comprising at least two and preferably at least four copies of an antigen from said at least one pathogenic micro organism each of which comprises a first multimerization domain and a detectable marker which comprises a second multimerization domain, wherein said complex is formed via the interaction of said first and said second multimerization domains, and
the amount of said detection reagent in association with said first and said second immunocomplexes is determined quantitatively and compared.

**8.** A detection reagent, comprising:

an antigenic peptide comprising at least one antigen from a pathogenic micro organism and a first multimerization domain;
a detectable marker and a second multimerization domain;
wherein said detection reagent comprises at least two and preferably at least four antigenic peptides conjugated to said detectable marker via the interaction of said first and said second multimerization domains.

**9.** The detection reagent of claim 8, wherein said antigenic peptide and/or said detection reagent further comprise an affinity tag.

**10.** The detection reagent of claim 8 or 9, wherein said antigenic peptide comprises a hexahistidine affinity tag, at least one antigen selected from the group consisting of: a yellow fever virus ED3 domain polypeptide, a West Nile virus ED3 domain polypeptide, a Dengue virus ED3 domain polypeptide, a St Louis encephalitis virus ED3 domain polypeptide, a Murray Valley encephalitis virus ED3 domain polypeptide and a Japanese encephalitis virus ED3 domain polypeptide; and a first multimerization domain comprising a biotinylation site, and
said detectable marker comprises a Horseradish Peroxidase enzyme and a second multimerization domain comprising either avidin, strepavidin, neutravidin or a functional derivative thereof.

**11.** The detection reagent of claim 8 to 10, wherein the peptide sequence of said antigenic peptide is selected from the group consisting of: SEQ ID NO: 1; SEQ ID NO: 4; SEQ ID NO: 5; SEQ ID NO: 6; SEQ ID NO: 7; SEQ ID NO: 8; SEQ ID NO: 9; SEQ ID NO: 10.

**12.** A nucleic acid encoding the antigenic peptide or detectable marker according to claim 11.

**13.** A method of preparing a detection reagent according to anyone of claims 9 to 12, wherein it comprises the steps:

a). preparing an expression vector containing a sequence encoding said antigenic peptide as defined in anyone of claims 9 to 12;
b). transforming an appropriate *E. coli* strain, with said expression vector and culturing said transformed strains;
c). purifying said antigenic peptide from a cellular extract by affinity purification for said affinity tag, and refolding the antigenic peptide *in vitro.*
d). mixing said purified antigenic peptide with said detectable marker under conditions which permit the formation of a complex between said purified antigenic peptide and said detectable marker via said first and said second multimerization domains.

**14.** A kit for diagnosing and/or screening for anti-flavivirus antibodies in a subject comprising:

- a solid support sensitized with an Ig binding protein which is directed against a specific class of Ig molecules of the animal species under consideration,
- a detection reagent as specified in any one of claims 8 to 11,
- at least one positive control, preferably a reference serum from an infected individual and
- at least one negative control, preferably a reference serum from a non-infected individual.

**15.** The kit according to claim 14, **characterized in that** the Ig binding protein is selected in the group consisting of anti-IgM, anti-IgG and anti-IgA, and said detection reagent comprises a streptavidin binding tag, a flavivirus ED3 domain, streptavidin and the Horseradish Peroxidase enzyme.

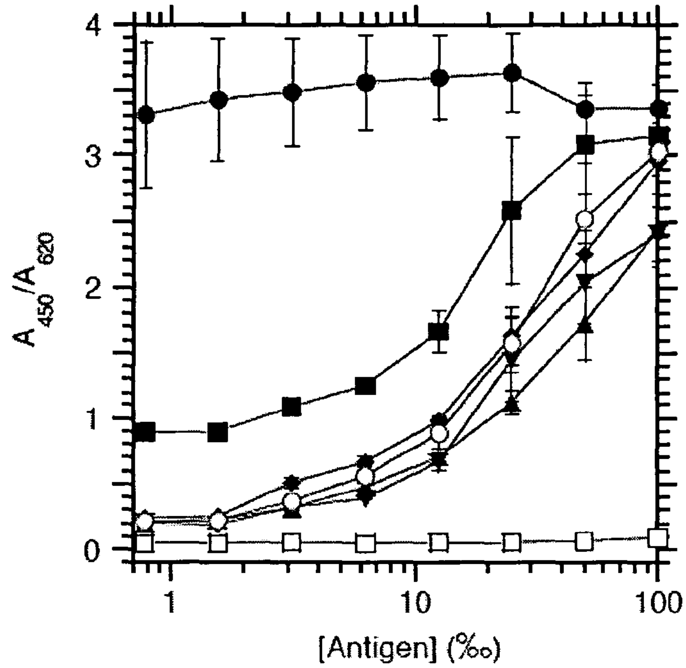


Figure 1

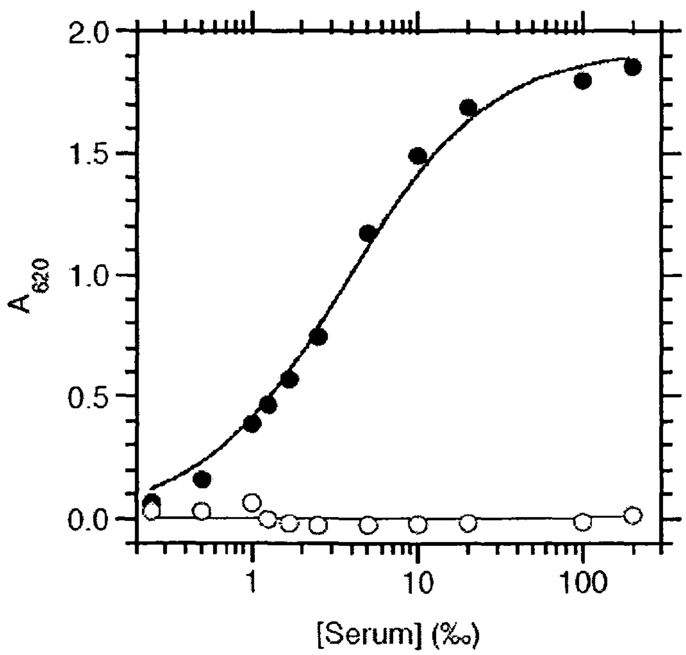


Figure 2

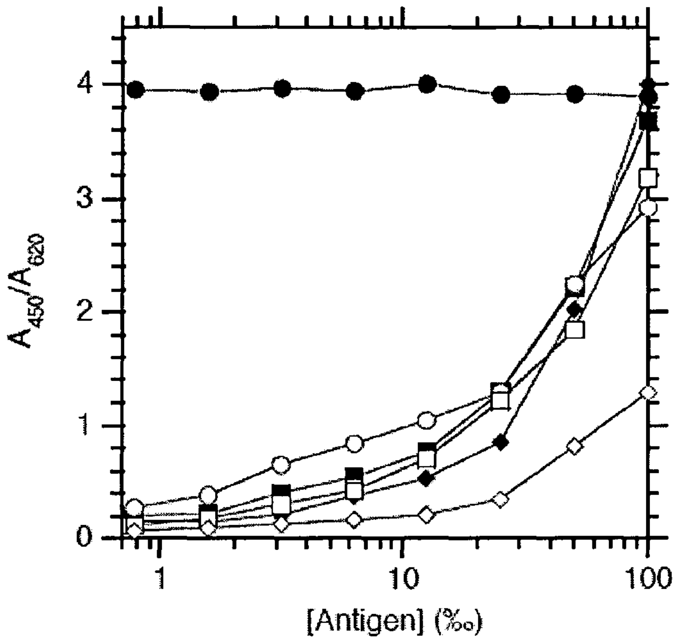


Figure 3

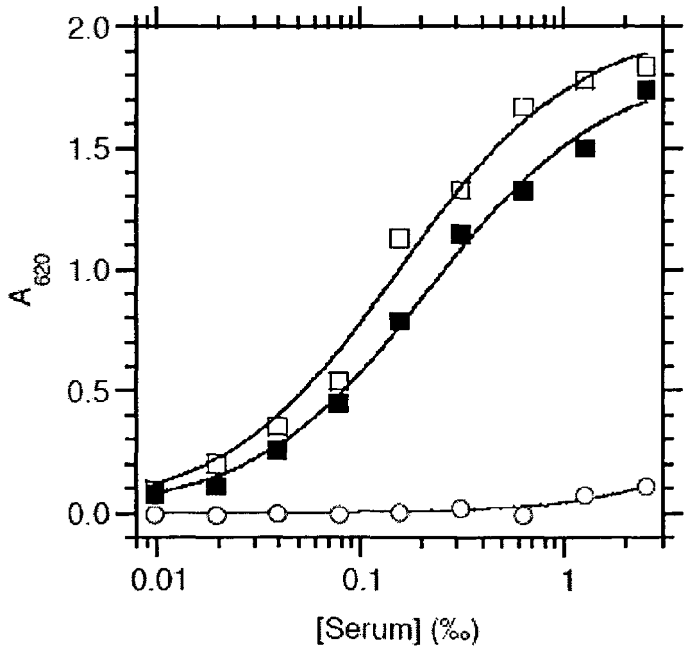


Figure 4

Europäisches Patentamt
European Patent Office
Office européen des brevets

# EUROPEAN SEARCH REPORT

Application Number
EP 09 29 0560

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | VILJA P ET AL: "Determination of immunoglobulin M antibodies for hepatitis B core antigen with a capture enzyme immunoassay and biotin-labeled core antigen produced in Escherichia coli." JOURNAL OF CLINICAL MICROBIOLOGY OCT 1985, vol. 22, no. 4, October 1985 (1985-10), pages 637-640, XP002557346 ISSN: 0095-1137 * abstract * ----- | 1-4,8 | INV. C07K14/18 G01N33/543 G01N33/68 |
| X | MORINET F ET AL: "Development of an IgM antibody capture test using labelled fusion protein as antigen for diagnosis of B19 human parvovirus infections" BEHRING INSTITUTE: MITTEILUNGEN, MARBURG, DE, vol. 85, 1 August 1990 (1990-08-01), pages 28-34, XP000196154 | 1-4,8 | |
| Y | * abstract * ----- | 6-7,15 | |
| X | LUDOLFS DIANA ET AL: "Highly specific detection of antibodies to tick-borne encephalitis (TBE) virus in humans using a domain III antigen and a sensitive immune complex (IC) ELISA." JOURNAL OF CLINICAL VIROLOGY : THE OFFICIAL PUBLICATION OF THE PAN AMERICAN SOCIETY FOR CLINICAL VIROLOGY JUN 2009, vol. 45, no. 2, June 2009 (2009-06), pages 125-128, XP002557347 ISSN: 1873-5967 * abstract * * page 126 * ----- | 1-5,8-9 | TECHNICAL FIELDS SEARCHED (IPC) C07K G01N |

-/--

The present search report has been drawn up for all claims

| Place of search | Date of completion of the search | Examiner |
|---|---|---|
| Munich | 1 December 2009 | Schalich, Juliane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

Europäisches Patentamt
European Patent Office
Office européen des brevets

# EUROPEAN SEARCH REPORT

Application Number
EP 09 29 0560

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2008/152528 A (PASTEUR INSTITUT [FR]; CENTRE NAT RECH SCIENT [FR]; BEDOUELLE HUGUES [) 18 December 2008 (2008-12-18)<br>* pages 9-18; claims 1-28; examples 1-10; sequences 1,8,10 * | 1-5,8-14 | |
| A | -& DATABASE Geneseq [Online]<br>2 April 2009 (2009-04-02), "DEN4-PhoA H6-ED3 hybrid protein, SEQ ID NO:8."<br>XP002557348<br>retrieved from EBI accession no. GSP:AUM00824<br>Database accession no. AUM00824<br>* abstract; compound * | 1-15 | |
| A | -& DATABASE Geneseq [Online]<br>2 April 2009 (2009-04-02), "DEN1-PhoA H6-ED3 coding sequence, SEQ ID NO:1."<br>XP002557349<br>retrieved from EBI accession no. GSN:AUM00817<br>Database accession no. AUM00817<br>* compound * | 1-15 | TECHNICAL FIELDS SEARCHED (IPC) |
| A | -& DATABASE Geneseq [Online]<br>2 April 2009 (2009-04-02), "YF-PhoA H6-ED3 hybrid protein, SEQ ID NO:10."<br>XP002557350<br>retrieved from EBI accession no. GSP:AUM00828<br>Database accession no. AUM00828<br>* compound *<br>-----<br>-/-- | 1-15 | |

The present search report has been drawn up for all claims

2

| Place of search | Date of completion of the search | Examiner |
|---|---|---|
| Munich | 1 December 2009 | Schalich, Juliane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 09 29 0560

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2006/078657 A2 (VAXINNATE CORP [US]; POWELL THOMAS J [US]; NAKAAR VALERIAN [US]; SONG) 27 July 2006 (2006-07-27) | 8-13 | |
| Y | * sequences 7, 160,162, 164, 166, 177 * * page 14 * * page 54 * * page 65 * * page 72 * * page 74, column 75 * * figures 50,51 * * table 2 * | 15 | |
| A | -& DATABASE Geneseq [Online] 21 September 2006 (2006-09-21), "Dengue 2 virus envelope protein." XP002557351 retrieved from EBI accession no. GSP:AEJ39112 Database accession no. AEJ39112 * abstract; compound * | 1-15 | |
| A | -& DATABASE Geneseq [Online] 21 September 2006 (2006-09-21), "Dengue 4 virus envelope protein." XP002557352 retrieved from EBI accession no. GSP:AEJ39116 Database accession no. AEJ39116 * abstract; compound * | 1-15 | TECHNICAL FIELDS SEARCHED (IPC) |
| A | -& DATABASE Geneseq [Online] 21 September 2006 (2006-09-21), "West Nile Virus envelope protein fragment, SEQ ID NO:7." XP002557353 retrieved from EBI accession no. GSP:AEJ38957 Database accession no. AEJ38957 * abstract; compound * | 1-15 | |
| | -/-- | | |

The present search report has been drawn up for all claims

| Place of search | Date of completion of the search | Examiner |
|---|---|---|
| Munich | 1 December 2009 | Schalich, Juliane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

Europäisches Patentamt
European Patent Office
Office européen des brevets

# EUROPEAN SEARCH REPORT

Application Number
EP 09 29 0560

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | -& DATABASE Geneseq [Online] 21 September 2006 (2006-09-21), "Japanese encephalitis virus envelope protein region, SEQ ID NO:177." XP002557354 retrieved from EBI accession no. GSP:AEJ39127 Database accession no. AEJ39127 * abstract; compound * | 1-15 | |
| A | -& DATABASE Geneseq [Online] 21 September 2006 (2006-09-21), "Dengue 3 virus envelope protein." XP002557355 retrieved from EBI accession no. GSP:AEJ39114 Database accession no. AEJ39114 * abstract; compound * ----- | 1-15 | |
| Y | US 2006/276622 A1 (DE LEYS ROBERT [BE]) 7 December 2006 (2006-12-07) * example 11; table 8 * ----- | 6 | TECHNICAL FIELDS SEARCHED (IPC) |
| Y | RIVETZ B ET AL: "New dengue antibody assay with unique differential detection of IgG and IgM antibodies" CLINICAL BIOCHEMISTRY, ELSEVIER INC, US, CA, vol. 42, no. 3, 1 February 2009 (2009-02-01), pages 180-184, XP025884895 ISSN: 0009-9120 [retrieved on 2008-11-06] * page 181 * ----- | 7 | |

The present search report has been drawn up for all claims

| Place of search | Date of completion of the search | Examiner |
|---|---|---|
| Munich | 1 December 2009 | Schalich, Juliane |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 09 29 0560

This annex lists the patent family members relating to the patent documents cited in the above-mentioned European search report.
The members are as contained in the European Patent Office EDP file on
The European Patent Office is in no way liable for these particulars which are merely given for the purpose of information.

01-12-2009

| Patent document cited in search report | Publication date | Patent family member(s) | Publication date |
|---|---|---|---|
| WO 2008152528 A | 18-12-2008 | EP 2003144 A1 | 17-12-2008 |
| WO 2006078657 A2 | 27-07-2006 | AR 052195 A1 | 07-03-2007 |
| | | AU 2006206647 A1 | 27-07-2006 |
| | | BR PI0606479 A | 11-03-2008 |
| | | CA 2594612 A1 | 27-07-2006 |
| | | EP 1841785 A2 | 10-10-2007 |
| | | JP 2008527009 T | 24-07-2008 |
| | | US 2008063657 A1 | 13-03-2008 |
| US 2006276622 A1 | 07-12-2006 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008152528 A, Bedouelle
- WO 2008152528 A2


### Non-patent literature cited in the description


- **AnandaRao, R. ; Swaminathan, S. ; Fernando, S. ; Jana, A.M. ; Khanna, N.** A custom-designed recombinant multiepitope protein as a dengue diagnostic reagent. *Protein Expr Purif,* 2005, vol. 41, 136-147
- **Barker, D.F. ; Campbell, A.M.** The birA gene of Escherichia coli encodes a biotin holoenzyme synthetase. *J Mol Biol,* 1981, vol. 146, 451-467
- **Beasley, D.W. ; Holbrook, M.R. ; Travassos Da Rosa, A.P. ; Coffey, L. ; Carrara, A.S. ; Phillippi-Falkenstein, K. ; Bohm, R.P., Jr. ; Ratterree, M.S. ; Lillibridge, K.M. ; Ludwig, G.V. et al.** Use of a recombinant envelope protein subunit antigen for specific serological diagnosis of West Nile virus infection. *J Clin Microbiol,* 2004, vol. 42, 2759-2765
- **Beckett, D. ; Kovaleva, E. ; Schatz, P.J.** A minimal peptide substrate in biotin holoenzyme synthetase-catalyzed biotinylation. *Protein Sci,* 1999, vol. 8, 921-929
- **Binz, H.K. ; Amstutz, P. ; Kohl, A. ; Stumpp, M.T. ; Briand, C. ; Forrer, P. ; Grutter, M.G. ; Pluckthun, A.** High-affinity binders selected from designed ankyrin repeat protein libraries. *Nat Biotechnol,* 2004, vol. 22, 575-582
- **Blondel A ; Bedouelle H.** Engineering the quaternary structure of an exported protein with a leucine zipper. *Protein Eng.,* 1991, vol. 4, 457-461
- **Bullock, W.O. ; Fernandez, J.M. ; Short, J.M.** XLI-Blue: a high efficiency plasmid transforming recA Escherichia coli strain with beta-galactosidase selection. *BioTechniques,* 1987, vol. 5, 376-379
- **Crill, W.D. ; Chang, G.J.** Localization and characterization of flavivirus envelope glycoprotein cross-reactive epitopes. *J Virol,* 2004, vol. 78, 13975-13986
- **Crill, W.D. ; Roehrig, J.T.** Monoclonal antibodies that bind to domain III of dengue virus E glycoprotein are the most efficient blockers of virus adsorption to Vero cells. *J Virol,* 2001, vol. 75, 7769-7773
- **Dauphin, G. ; Zientara, S.** West Nile virus: recent trends in diagnosis and vaccine development. *Vaccine,* 2007, vol. 25, 5563-5576
- **de Oliveira Poersch, C. ; Pavoni, D.P. ; Queiroz, M.H. ; de Borba, L. ; Goldenberg, S. ; dos Santos, C.N. ; Krieger, M.A.** Dengue virus infections: comparison of methods for diagnosing the acute disease. *J Clin Virol,* 2005, vol. 32, 272-277
- **Dussart, P. ; Labeau, B. ; Lagathu, G. ; Louis, P. ; Nunes, M.R. ; Rodrigues, S.G. ; Storck-Herrmann, C. ; Cesaire, R. ; Morvan, J. ; Flamand, M. et al.** Evaluation of an enzyme immunoassay for detection of dengue virus NS1 antigen in human serum. *Clin Vaccine Immunol,* 2006, vol. 13, 1185-1189
- **Dussart, P. ; Petit, L. ; Labeau, B. ; Bremand, L. ; Leduc, A. ; Moua, D. ; Matheus, S. ; Baril, L.** Evaluation of Two New Commercial Tests for the Diagnosis of Acute Dengue Virus Infection Using NS1 Antigen Detection in Human Serum. *PLoS Negl Trop Dis,* 2008, vol. 2, e280
- **Gould, E.A. ; Solomon, T.** Pathogenic flaviviruses. *Lancet,* 2008, vol. 371, 500-509
- **Gritsun, T.S. ; Holmes, E.C. ; Gould, E.A.** Analysis of flavivirus envelope proteins reveals variable domains that reflect their antigenicity and may determine their pathogenesis. *Virus Res,* 1995, vol. 35, 307-321
- **Halstead, S.B.** Pathogenesis of dengue: challenges to molecular biology. *Science,* 1988, vol. 239, 476-481
- **Holbrook, M.R. ; Shope, R.E. ; Barrett, A.D.** Use of recombinant E protein domain III-based enzyme-linked immunosorbent assays for differentiation of tick-borne encephalitis serocomplex flaviviruses from mosquito-borne flaviviruses. *J Clin Microbiol,* 2004, vol. 42, 4101-4110
- **Holmes, D.A. ; Purdy, D.E. ; Chao, D.Y. ; Noga, A.J. ; Chang, G.J.** Comparative analysis of immunoglobulin M (IgM) capture enzyme-linked immunosorbent assay using virus-like particles or virus-infected mouse brain antigens to detect IgM antibody in sera from patients with evident flaviviral infections. *J Clin Microbiol,* 2005, vol. 43, 3227-3236
- **Igarashi, A.** Impact of dengue virus infection and its control. *FEMS Immunol Med Microbiol,* 1997, vol. 18, 291-300

- **Innis, B.L. ; Nisalak, A. ; Nimmannitya, S. ; Kusalerdchariya, S. ; Chongswasdi, V. ; Suntayakorn, S. ; Puttisri, P. ; Hoke, C.H.** An enzyme-linked immunosorbent assay to characterize dengue infections where dengue and Japanese encephalitis co-circulate. *Am J Trop Med Hyg,* 1989, vol. 40, 418-427
- **Johnson, A.J. ; Martin, D.A. ; Karabatsos, N. ; Roehrig, J.T.** Detection of anti-arboviral immunoglobulin G by using a monoclonal antibody-based capture enzyme-linked immunosorbent assay. *J Clin Microbiol,* 2000, vol. 38, 1827-1831
- **Kanai, R. ; Kar, K. ; Anthony, K. ; Gould, L.H. ; Ledizet, M. ; Fikrig, E. ; Marasco, W.A. ; Koski, R.A. ; Modis, Y.** Crystal structure of west nile virus envelope glycoprotein reveals viral surface epitopes. *J Virol,* 2006, vol. 80, 11000-11008
- **Kao, C.L. ; King, C.C. ; Chao, D.Y. ; Wu, H.L. ; Chang, G.J.** Laboratory diagnosis of dengue virus infection: current and future perspectives in clinical diagnosis and public health. *J Microbiol Immunol Infect,* 2005, vol. 38, 5-16
- **Kuno, G.** Serodiagnosis of flaviviral infections and vaccinations in humans. *Adv Virus Res,* 2003, vol. 61, 3-65
- **Lanciotti, R.S. ; Calisher, C.H. ; Gubler, D.J. ; Chang, G.J. ; Vorndam, A.V.** Rapid detection and typing of dengue viruses from clinical samples by using reverse transcriptase-polymerase chain reaction. *J Clin Microbiol,* 1992, vol. 30, 545-551
- **Lisova, O. ; Hardy, F. ; Petit, V. ; Bedouelle, H.** Mapping to completeness and transplantation of a group-specific, discontinuous, neutralizing epitope in the envelope protein of dengue virus. *J Gen Virol,* 2007, vol. 88, 2387-2397
- **Ludolfs, D. ; Schilling, S. ; Altenschmidt, J. ; Schmitz, H.** Serological differentiation of infections with dengue virus serotypes 1 to 4 by using recombinant antigens. *J Clin Microbiol,* 2002, vol. 40, 4317-4320
- **Makino, Y. ; Tadano, M. ; Saito, M. ; Maneekarn, N. ; Sittisombut, N. ; Sirisanthana, V. ; Poneprasert, B. ; Fukunaga, T.** Studies on serological cross-reaction in sequential flavivirus infections. *Microbiol Immunol,* 1994, vol. 38, 951-955
- **Martin, D.A. ; Biggerstaff, B.J. ; Allen, B. ; Johnson, A.J. ; Lanciotti, R.S. ; Roehrig, J.T.** Use of immunoglobulin m cross-reactions in differential diagnosis of human flaviviral encephalitis infections in the United States. *Clin Diagn Lab Immunol,* 2002, vol. 9, 544-549
- **Martin, D.A. ; Muth, D.A. ; Brown, T. ; Johnson, A.J. ; Karabatsos, N. ; Roehrig, J.T.** Standardization of immunoglobulin M capture enzyme-linked immunosorbent assays for routine diagnosis of arboviral infections. *J Clin Microbiol,* 2000, vol. 38, 1823-1826
- **Mathew, A. ; Rothman, A.L.** Understanding the contribution of cellular immunity to dengue disease pathogenesis. *Immunol Rev,* 2008, vol. 225, 300-313
- **Modis, Y. ; Ogata, S. ; Clements, D. ; Harrison, S.C.** A ligand-binding pocket in the dengue virus envelope glycoprotein. *Proc Natl Acad Sci U S A,* 2003, vol. 100, 6986-6991
- **Modis, Y. ; Ogata, S. ; Clements, D. ; Harrison, S.C.** Structure of the dengue virus envelope protein after membrane fusion. *Nature,* 2004, vol. 427, 313-319
- **Modis, Y. ; Ogata, S. ; Clements, D. ; Harrison, S.C.** Variable surface epitopes in the crystal structure of dengue virus type 3 envelope glycoprotein. *J Virol,* 2005, vol. 79, 1223-1231
- **Mongkolsapaya, J. ; Dejnirattisai, W. ; Xu, X.N. ; Vasanawathana, S. ; Tangthawornchaikul, N. ; Chairunsri, A. ; Sawasdivom, S. ; Duangchinda, T. ; Dong, T. ; Rowland-Jones, S. et al.** Original antigenic sin and apoptosis in the pathogenesis of dengue hemorrhagic fever. *Nat Med,* 2003, vol. 9, 921-927
- **Mukhopadhyay, S. ; Kuhn, R.J. ; Rossmann, M.G.** A structural perspective of the flavivirus life cycle. *Nat Rev Microbiol,* 2005, vol. 3, 13-22
- **Müller KM ; Arndt KM ; Alber T.** Protein fusions to coiled-coil domains. *Methods Enzymol.,* 2000, vol. 328, 261-282
- **Pack P ; Plückthun A.** Miniantibodies: use of amphipathic helices to produce functional, flexibly linked dimeric FV fragments with high avidity in Escherichia coli. *Biochemistry,* 1992, vol. 31, 1579-1584
- **Potts, J.A. ; Rothman, A.L.** Clinical and laboratory features that distinguish dengue from other febrile illnesses in endemic populations. *Trop Med Int Health,* 2008, vol. 13, 1328-1340
- **Rey, F.A. ; Heinz, F.X. ; Mandl, C. ; Kunz, C. ; Harrison, S.C.** The envelope glycoprotein from tick-borne encephalitis virus at 2 A resolution. *Nature,* 1995, vol. 375, 291-298
- **Rice, P. ; Longden, I. ; Bleasby, A.** EMBOSS: the European Molecular Biology Open Software Suite. *Trends Genet,* 2000, vol. 16, 276-277
- **Sambrook, J. ; Russell, D.W.** Molecular Cloning: A Laboratory Manual. Cold Spring Harbor Press, 2001
- **Sanchez, M.D. ; Pierson, T.C. ; McAllister, D. ; Hanna, S.L. ; Puffer, B.A. ; Valentine, L.E. ; Murtadha, M.M. ; Hoxie, J.A. ; Doms, R.W.** Characterization of neutralizing antibodies to West Nile virus. *Virology,* 2005, vol. 336, 70-82
- **Schilling, S. ; Ludolfs, D. ; Van An, L. ; Schmitz, H.** Laboratory diagnosis of primary and secondary dengue infection. *J Clin Virol,* 2004, vol. 31, 179-184

- **Schwartz, E. ; Mileguir, F. ; Grossman, Z. ; Mendelson, E.** Evaluation of ELISA-based sero-diagnosis of dengue fever in travelers. *J Clin Virol,* 2000, vol. 19, 169-173
- **Schmidt TGM ; Skerra A.** The Strep-tag system for one-step purification and high-affinity detection or capturing of proteins. *NATURE PROTOCOLS,* 2007, vol. 2, 1528-1535
- **Shu, P.Y. ; Huang, J.H.** Current advances in dengue diagnosis. *Clin Diagn Lab Immunol,* 2004, vol. 11, 642-650
- **Simmons, M. ; Porter, K.R. ; Escamilla, J. ; Graham, R. ; Watts, D.M. ; Eckels, K.H. ; Hayes, C.G.** Evaluation of recombinant dengue viral envelope B domain protein antigens for the detection of dengue complex-specific antibodies. *Am J Trop Med Hyg,* 1998, vol. 58, 144-151
- **Takasaki, T. ; Nawa, M. ; Yamada, K.I. ; Harada, M. ; Takeda, A. ; Kurane, I.** Evaluation of dengue IgM detection tests using sera from patients with autoimmune diseases. *J Virol Methods,* 2002, vol. 102, 61-66
- **Teles, F.R. ; Prazeres, D.M. ; Lima-Filho, J.L.** Trends in dengue diagnosis. *Rev Med Virol,* 2005, vol. 15, 287-302
- **Weber, P.C. ; Ohlendorf, D.H. ; Wendoloski, J.J. ; Salemme, F.R.** Structural origins of high-affinity biotin binding to streptavidin. *Science,* 1989, vol. 243, 85-88
- **Westaway, E.G. ; Shew, M. ; Della-Porta, A.J.** Reactions of purified hemagglutinating antigens of flaviviruses with 19S and 7S antibodies. *Infect Immun,* 1975, vol. 11, 630-634
- **Wilchek, M. ; Bayer, E.A. ; Livnah, O.** Essentials of biorecognition: the (strept)avidin-biotin system as a model for protein-protein and protein-ligand interaction. *Immunol Lett,* 2006, vol. 103, 27-32
- **Yamada, K. ; Nawa, M. ; Takasaki, T. ; Yabe, S. ; Kurane, I.** Laboratory diagnosis of dengue virus infection by reverse transcriptase polymerase chain reaction (RT-PCR) and IgM-capture enzyme-linked immunosorbent assay (ELISA). *Jpn J Infect Dis,* 1999, vol. 52, 150-155
- **Zhang J ; Tanha J ; Hirama T ; Khieu NH ; To R ; Tong-Sevinc H ; Stone E ; Brisson JR ; MacKenzie CR.** Pentamerization of single-domain antibodies from phage libraries: a novel strategy for the rapid generation of high-avidity antibody reagents. *J Mol Biol.,* 2004, vol. 335, 49-56